# EUROPEAN PATENT APPLICATION

(11) **EP 4 289 861 A1**
(43) Date of publication of application: **13.12.2023**
(21) Application number: 21924079.3
(22) Date of filing: 17.06.2021
(51) Int. Cl.: C07K 16/24, C12N 15/13, A61K 39/395, A61P 37/06

(54) **ANTIBODIES AGAINST HUMAN TSLP AND USE THEREOF**

(30) Priority: 03.02.2021 CN 202110147687
(71) Applicant: Beijing Wisdomab Biotechnology Co., Ltd, Beijing 101111 (CN); Genrix(Shanghai) Biopharmaceutical Co., Ltd., Shanghai 201203 (CN); Chongqing Genrix Biopharmaceutial Co., Ltd., Chongqing 401319 (CN)
(72) Inventor: ZHANG, Xueping, Beijing (CN); LIU, Zhigang, Beijing (CN); ZHOU, Xiaowei, Beijing (CN); HU, Junjie, Beijing (CN); HAO, Xiaobo, Beijing (CN); LIU, Yulan, Beijing (CN)
(74) Representative: Nederlandsch Octrooibureau
(86) International application number: PCT/CN2021/100657
(87) International publication number: WO 2022/166072

(57) **Abstract**

A bispecific antibody and a monoclonal antibody against human thymic stromal lymphopoietin (TSLP), and a use thereof.

## Description

### CROSS REFERENCE TO RELATED APPLICATION

The present application claims priority to Chinese Patent Application No. 202110147687.1 filed on February 3, 2021, which is incorporated herein by reference in its entirety.

### TECHNICAL FIELD

The present application generally relates to the field of genetic engineering and antibody medicine. In particular, the present application relates to a bispecific antibody and a monoclonal antibody against human TSLP, and uses thereof.

### BACKGROUND

Human thymic stromal lymphopoietin (TSLP), also known as IL-7-like cytokine ^{[1]}, is a member of IL-2 cytokine family. TSLP is secreted predominantly by epithelial cells of thymus, lung, intestine and skin ^{[2, 3]}, and to a lesser extent by some fibroblasts, airway smooth muscle endothelial cells, mast cells, monocytes, granulocytes and dendritic cells (DC)^{[4-14]}. The expression of TSLP is regulated by some factors, such as allergens, proinflammatory factors (IL-1β, TNF-α, TGF-β, IL-4, and IL-13), Th2 cytokine, trauma, mechanical injury and bacteria, etc ^{[15-18]}.

The target cells of TSLP co-express TSLP receptor (thymic stromal lymphopoietin receptor, TSLPR) and IL-7 receptor α chain (IL-7Rα) ^{[19]}. TSLPR binds to TSLP with high affinity, thereby allosterically activating TSLP, which in turn recruits IL-7Rα to form TSLP-TSLPR-IL-7Rα ternary complex that can transmit signals. IL-7Rα binds to TSLP-TSLPR with high affinity, but binds to TSLP very weakly ^{[20]}.

Asthma is a chronic disease characterized by inflammation of the airways, and is clinically characterized by recurrent symptoms such as wheezing, shortness of breath, chest tightness and coughing ^{[21]}. Globally, about 300 million people suffer from asthma ^{[22]}. The global prevalence of asthma has been increasing at a rate of about 1% every year for the past two decades. The existing therapeutic drugs, including bronchodilators, glucocorticoids, combined preparations of bronchodilators and glucocorticoids (such as seretide and symbicort), leukotriene modulators, long-acting cholinergic receptor antagonists (such as tiotropium bromide), and IgE antibodies, cannot control the condition of all asthmatics.

Numerous studies have demonstrated that about 2/3 of severe asthma is characterized by overexpression of Th2 cytokines, and TSLP is an important factor causing overexpression of Th2 cytokines. The TSLP-TSLPR effect is mainly mediated through JAK-STAT signaling pathway ^{[2]}. Research suggests that upregulated TSLP will bind to TSLPR on DC cells so as to activate JAK, recruit transcription factor STATS, and cause downstream signaling, and ultimately leading to DC cell activation. DC cell activation exhibits upregulated expression of co-stimulatory molecules (such as CD80, CD40 and CD86) and secretion of chemokines (such as TARC/CCL17, MDC/CCL22 and I-309/CCL1), which provides a favorable microenvironment for the differentiation of Th0 to Th2 cells to direct Th2 cell-predominant inflammatory response accompanied by release of factors (IL-4, IL-13 and IL-5) ^{[11, 12, 23, 24]}. TSLP transgenic mice are susceptible to specific antigens and thus develop asthma, whereas symptoms are significantly reduced in TSLP receptor knockout mice ^{[24]}. Analysing the mechanisms of asthma and inflammation, anti-cytokine (IL-4, IL-13, IL-5) drugs only target specific inflammatory molecules that drive asthma and inflammation, and are only suitable for certain types of severe asthma patients, i.e., subgroup patients, such as eosinophilic asthma. TSLP is significantly different from targets such as IL-4 and IL-5. TSLP is active in the early upstream of inflammatory cascade, and may be suitable for a wide range of patients with severe uncontrolled asthma.

Researchers have conducted extensive exploration and research on drugs targeting TSLP. Anti-TSLP monoclonal antibodies can effectively block TSLP/TSLPR action, reverse airway inflammation, prevent tissue structural changes, and reduce airway hyperresponsiveness (AHR) and TGF-β1 level in a mite dust-induced mouse model of asthma ^{[25]}. In a serum protein-induced mouse model of asthma, anti-TSLP monoclonal antibodies effectively reduce the expression of Th2 factors (such as IL-4 and IL-5) ^{[26]}. The safety of anti-TSLP monoclonal antibodies has also been well established in monkeys. Moreover, the only clinically investigated anti-TSLP monoclonal antibody around the world shows a good objective response rate in the early clinical phase, effectively alleviating the symptoms of the subjects ^{[27]}

Bispecific antibody (BsAb) is a class of artificial antibody which comprises two different antigen binding sites, can build a bridge between target cells and functional molecules (cells) and stimulate a directed immune response. It has now become a research hotspot in the field of antibody engineering and has a broad application prospect in the immunotherapy of diseases.

The development and application of new anti-TSLP antibodies are needed in this field. Therefore, in view of the wide applicability of anti-TSLP antibodies and due to clinical needs, it is of great biological and medical significance to explore and develop new anti-TSLP antibodies.

### SUMMARY OF THE INVENTION

In a first aspect, there is provided in the present application a bispecific antibody comprising a first binding fragment and a second binding fragment that bind to non-overlapping epitopes of human thymic stromal lymphopoietin (TSLP).

In some embodiments of the first aspect, the bispecific antibody is capable of blocking the binding of human TSLP to human TSLP receptor (TSLPR), and the bispecific antibody is capable of blocking the binding of human IL-7 receptor alpha chain (IL-7Rα) to human TSLP: TSLPR complex.

In some embodiments of the first aspect, the epitope of human TSLP bound by one of the first binding fragment and the second binding fragment partially overlaps with the epitope of human TSLP bound by IL-7Rα.

In some embodiments of the first aspect, the first binding fragment comprises HCDR1 as set forth in SEQ ID NO:29, HCDR2 as set forth in SEQ ID NO:30, HCDR3as set forth in SEQ ID NO:31, LCDR1 as set forth in SEQ ID NO:32, LCDR2 as set forth in SEQ ID NO:33, and LCDR3 as set forth in SEQ ID NO:34; wherein the amino acid sequences of HCDRs and LCDRs are defined according to Kabat.

In some embodiments of the first aspect, the second binding fragment comprises HCDR1 as set forth in SEQ ID NO:35, HCDR2 as set forth in SEQ ID NO:36, HCDR3 as set forth in SEQ ID NO:37, LCDR1 as set forth in SEQ ID NO:32, LCDR2 as set forth in SEQ ID NO:33, and LCDR3 as set forth in SEQ ID NO:34; wherein the amino acid sequences of HCDRs and LCDRs are defined according to Kabat.

In some embodiments of the first aspect, the amino acid sequence of the heavy chain variable region of the first binding fragment is set forth in SEQ ID NO: 24, and the amino acid sequence of the light chain variable region of the first binding fragment is set forth in SEQ ID NO: 21; or
the amino acid sequence of the heavy chain variable region of the first binding fragment is set forth in SEQ ID NO: 24, and the amino acid sequence of the light chain variable region of the first binding fragment is set forth in SEQ ID NO: 28.

In some embodiments of the first aspect, the amino acid sequence of the heavy chain variable region of the second binding fragment is set forth in SEQ ID NO: 20, and the amino acid sequence of the light chain variable region of the second binding fragment is set forth in SEQ ID NO: 21; or
the amino acid sequence of the heavy chain variable region of the second binding fragment is set forth in SEQ ID NO: 20, and the amino acid sequence of the light chain variable region of the second binding fragment is set forth in SEQ ID NO: 28.

In some embodiments of the first aspect, the bispecific antibody is an IgG1 antibody comprising a first heavy chain constant region and a second heavy chain constant region, wherein the amino acids at positions 354 and 366 of the first heavy chain constant region are C and W, respectively, and the amino acids at positions 349, 366, 368 and 407 of the second heavy chain constant region are C, S, A and V, respectively; and the amino acid positions of antibody constant regions are determined according to EU numbering.

In some embodiments of the first aspect, the bispecific antibody is an IgG1 antibody comprising a first heavy chain constant region and a second heavy chain constant region, wherein the amino acids at positions 234, 235 and 331 of the first heavy chain constant region and the second heavy chain constant region are F, E and S, respectively; and the amino acid positions of antibody constant regions are determined according to EU numbering.

In some embodiments of the first aspect, the forms of the first binding fragment and the second binding fragment are independently selected from a single-chain fragment variable (scFv) or a Fab fragment.

In some embodiments of the first aspect, the first binding fragment and the second binding fragment have the same light chain variable region.

In some embodiments of the first aspect, the bispecific antibody comprises the heavy chain as set forth in SEQ ID NO:38 and the light chain as set forth in one of SEQ ID NOs: 39 and 40.

In some embodiments of the first aspect, the bispecific antibody comprises the heavy chain as set forth in SEQ ID NO:41 and the light chain as set forth in one of SEQ ID NOs: 39 and 40.

In a second aspect, there is provided in the present application a monoclonal antibody that binds to human TSLP, comprising HCDR1 as set forth in SEQ ID NO:29, HCDR2 as set forth in SEQ ID NO:30, HCDR3 as set forth in SEQ ID NO:31, LCDR1 as set forth in SEQ ID NO:32, LCDR2 as set forth in SEQ ID NO:33, and LCDR3 as set forth in SEQ ID NO:34; wherein the amino acid sequences of HCDRs and LCDRs are defined according to Kabat.

In a third aspect, there is provided in the present application a pharmaceutical composition comprising the bispecific antibody of the first aspect, or the monoclonal antibody of the second aspect, and a pharmaceutically acceptable excipient, diluent, or carrier.

In a fourth aspect, there is provided in the present application use of the bispecific antibody of the first aspect, the monoclonal antibody of the second aspect, or the pharmaceutical composition of the third aspect in the manufacture of a medicament for the prevention or treatment of TSLP-mediated diseases.

### BRIEF DESCRIPTION OF THE DRAWINGS

Figure 1 shows the results from an ELISA assay assessing the inhibitory effect of the anti-human TSLP monoclonal antibody S17C7+L3E8-M1 on the binding of TSLP to TSLPR.
Figure 2 shows the results from an ELISA assay assessing the ability of the anti-human TSLP monoclonal antibody S17C7+L3E8-M1 to inhibit the binding of Tezepelumab-mIgG2a to TSLP.
Figure 3 shows the results from a chemiluminescent cell viability assay assessing the ability of the anti-human TSLP monoclonal antibody S17C7+L3E8-M1 to inhibit the proliferation-promoting effect of TSLP on BAF3-TSLPR/IL-7Rα cells.
Figure 4 shows the results from an ELISA assay assessing the ability of the anti-human TSLP monoclonal antibody S17C7+L3E8-M1 to inhibit the secretion of TRAC by PBMCs stimulated by TSLP.
Figure 5 shows the inhibitory effect of the humanized anti-human TSLP monoclonal antibody H3B10-M1+L3E8-M1 on the binding of TSLP to TSLPR.
Figure 6 shows the results from a chemiluminescent cell viability assay assessing the abilities of anti-human TSLP monoclonal antibodies and two monoclonal antibodies in combination to inhibit the proliferation-promoting effect of TSLP on BAF3-TSLPR/IL-7Rα cells.
Figure 7 shows the results from an ELISA assay assessing the abilities of anti-human TSLP monoclonal antibodies and two monoclonal antibodies in combination to inhibit the secretion of TRAC by PBMCs stimulated by TSLP.
Figure 8 shows the results from an ELISA assay assessing the inhibitory effects of anti-human TSLP bispecific antibodies on the binding of TSLP to TSLPR.
Figure 9 shows the results from a chemiluminescent cell viability assay assessing the abilities of anti-human TSLP bispecific antibodies to inhibit the proliferation-promoting effect of TSLP on BAF3-TSLPR/IL-7Rα cells.
Figure 10 shows the results from an ELISA assay assessing the ability of anti-human TSLP bispecific antibody to inhibit the secretion of TARC by PBMCs stimulated by TSLP.
Figure 11 shows the results of analyzing the binding epitopes of the anti-human TSLP antibodies using Biacore T200, in which panels A-D respectively show the results of analysis by using H3B10-M1+L3E8-M7, H1A10+L3E8-M7, the bispecific antibody H1A10×H3B10-M1+L3E8-M7 and Tezepelumab as the first antibodies.
Figure 12 shows the structural of an antibody-antigen complex, in which the TSLP antigen is shown on the left and H1A10+L3E8-M1 Fab is shown on the right.
Figure 13 shows the results of assessing the abilities of anti-human TSLP antibodies to block the binding of human TSLPR to human TSLP using Biacore T200.
Figure 14 shows the results of assessing the abilities ofanti-human TSLP antibodies to block the binding of human IL-7Rα to human TSLP: TSLPR complex using Biacore T200.

### DESCRIPTION OF THE SEQUENCES

SEQ ID NO: 1 shows the amino acid sequence of the long version of the extracellular domain of human (*homo sapiens*) TSLP(hTSLP1-ECD).
SEQ ID NO: 2 shows the amino acid sequence of the short version of the extracellular domain of human (*homo sapiens*) TSLP (hTSLP2-ECD).
SEQ ID NO: 3 shows the amino acid sequence of the extracellular domain of mouse (*mus musculus*) TSLP (mTSLP-ECD).
SEQ ID NO: 4 shows the amino acid sequence of the extracellular domain of cynomolgus monkey (*Macacafascicularis*) TSLP (mfTSLP1-ECD).
SEQ ID NO: 5 shows the amino acid sequence of hTSLP1 mutant (hTSLP1-m) with the deletion of furin recognition site.
SEQ ID NO: 6 shows the amino acid sequences of hTSLP2 mutant (hTSLP2-m) with the deletion of furin recognition site.
SEQ ID NO: 7 shows the amino acid sequence of mfTSLP mutant (mfTSLP1-m) with the deletion of furin recognition site.
SEQ ID NO: 8 shows the amino acid sequence of His tag.
SEQ ID NO:9 shows the amino acid sequence of the Fc fragment (hFc) of human (*homo sapiens*) IgG1 antibody.
SEQ ID NO: 10 shows the amino acid sequence of the Fc fragment (mFc) of mouse (*mus musculus*) IgG2a antibody.
SEQ ID NO: 11 shows the amino acid sequence of the heavy chain constant region of human (*homo sapiens*) IgG1 subtype.
SEQ ID NO: 12 shows the amino acid sequence of the heavy chain constant region of human (*homo sapiens*) IgG2 subtype.
SEQ ID NO: 13 shows the amino acid sequence of the heavy chain constant region of human (*homo sapiens*) IgG4 subtype.
SEQ ID NO: 14 shows the amino acid sequence of the heavy chain constant region of mouse (*mus musculus*) IgG1 subtype.
SEQ ID NO: 15 shows the amino acid sequence of the heavy chain constant region of mouse (*mus musculus*) IgG2a subtype.
SEQ ID NO: 16 shows the amino acid sequence of the light chain constant region of human (*homo sapiens*) kappa subtype.
SEQ ID NO: 17 shows the amino acid sequence of the light chain constant region of human (*homo sapiens*) lamda subtype.
SEQ ID NO: 18 shows the amino acid sequence of the light chain constant region of mouse (*mus musculus*) kappa subtype.
SEQ ID NO: 19 shows the amino acid sequence of the light chain constant region of mouse (*mus musculus*) lamda subtype.
SEQ ID NO:20 shows the amino acid sequence of the heavy chain variable region of the humanized antibody H1A10+L3E8-M1with the amino acid sequences of HCDR1-HCDR3 as set forth in SEQ ID NO:35, SEQ ID NO:36 and SEQ ID NO:37, respectively.
SEQ ID NO:21 shows the amino acid sequence of the light chain variable region L3E8-M1 of the humanized antibody H1A10+L3E8-M1 with the amino acid sequences of LCDR1-LCDR3 as set forth in SEQ ID NO:32, SEQ ID NO:33 and SEQ ID NO:34, respectively.
SEQ ID NO:22 shows the amino acid sequence of the heavy chain variable region of the anti-human TSLP control antibody Tezepelumab.
SEQ ID NO:23 shows the amino acid sequence of the light chain variable region of the anti-human TSLP control antibody Tezepelumab.
SEQ ID NO:24 shows the amino acid sequence of the humanized heavy chain mutant H3B10-M1 with the amino acid sequences of HCDR1-HCDR3 as set forth in SEQ ID NO:29, SEQ ID NO:30 and SEQ ID NO:31, respectively.
SEQ ID NO:25 shows the amino acid sequence of L3E8-M2.
SEQ ID NO:26 shows the amino acid sequence of L3E8-M3.
SEQ ID NO:27 shows the amino acid sequence of L3E8-M4.
SEQ ID NO:28 shows the amino acid sequence of the mutant L3E8-M7 with the amino acid sequences of LCDR1-LCDR3 as set forth in SEQ ID NO:32, SEQ ID NO:33 and SEQ ID NO:34, respectively.
SEQ ID NO:38 shows the amino acid sequence of H3B10-M1-IgG1m3-H.
SEQ ID NO:39 shows the amino acid sequence of the light chain comprising the L3E8-M1 light chain variable region.
SEQ ID NO:40 shows the amino acid sequence of the light chain comprising the L3E8-M7 light chain variable region.
SEQ ID NO:41 shows the amino acid sequence of H1A10-IgG1m3-K.
SEQ ID NO:42 shows the amino acid sequence of the extracellular domain (hTSLPR-ECD) of human (*homo sapiens*) TSLP receptor.
SEQ ID NO:43 shows the amino acid sequence of the extracellular domain (hIL-7Rα-ECD) of human (*homo sapiens*) IL-7 receptor alpha chain.

### DETAILED DESCRIPTION OF THE INVENTION

The present inventors have conducted extensive research and development of antibody drugs that bind to human TSLP, and have obtained novel bispecific antibodies and monoclonal antibodies that bind to human TSLP by antibody engineering techniques. In various aspects of the present application, there are provided novel bispecific and monoclonal antibodies that bind to human TSLP, polynucleotides encoding the bispecific or monoclonal antibodies, vectors comprising the polynucleotides, host cells comprising the polynucleotides or vectors, methods of preparing and purifying the bispecific or monoclonal antibodies, and medical and biological use of the bispecific or monoclonal antibodies. Based on the sequences of the variable regions of the bispecific or monoclonal antibodies provided herein, full-length bispecific or monoclonal antibody molecules can be constructed for use in the clinic as drugs for preventing or treating TSLP mediated diseases.

Unless otherwise indicated, the inventions of the present application can be implemented using conventional molecular biology, microbiology, cell biology, biochemistry, and immunological techniques in the art.

Unless otherwise indicated, the terms used in the present application have the meanings commonly understood by those skilled in the art.

### DEFINITIONS

As used herein, the term "antibody" refers to an immunoglobulin molecule that is capable of specifically binding to a target via at least one antigen recognition site located in a variable region of the immunoglobulin molecule. Targets include, but are not limited to, carbohydrates, polynucleotides, lipids, and polypeptides, etc. As used herein, an "antibody" includes not only an intact (i.e., full-length) antibody, but also a binding fragment thereof (e.g., Fab, Fab', F(ab')₂, Fv), a variant thereof, a fusion protein comprising portions of an antibody, a humanized antibody, a chimeric antibody, a diabody, a linear antibody, a single-chain fragment variable , a VHH antibody, a multi-specific antibody (e.g., a bispecific antibody), and any other modified configurations of an immunoglobulin molecule comprising a desired specific antigen recognition site, including a glycosylated variant of an antibody, an amino acid sequence variant of an antibody, and a covalently modified antibody.

Typically, an intact or full-length antibody comprises two heavy chains and two light chains. Each heavy chain contains a heavy chain variable region (VH) and first, second and third constant regions (CH1, CH2 and CH3). Each light chain contains a light chain variable region (VL) and a constant region (CL). A full-length antibody may be of any type of antibody, such as an IgD, IgE, IgG, IgA, or IgM (or their subtypes) antibody, but not necessarily belongs to any particular type. Immunoglobulins can be assigned to different types depending on their amino acid sequences of the heavy chain constant regions. Generally, immunoglobulins have five main types, i.e., IgA, IgD, IgE, IgG, and IgM, and some of these types can be further classified into subtypes (isotypes), such as IgG1, IgG2, IgG3, IgG4, IgA1, and IgA2. Heavy chain constant regions corresponding to individual immunoglobulin types are referred to as α, δ, ε, γ, and µ, respectively. Subunit structures and three-dimensional structures of different types of immunoglobulins are well known.

As used herein, the term "bispecific antibody" refers to an antibody that can simultaneously bind to two antigen epitopes. The two antigen epitopes may be on different antigens or on the same antigen. A bispecific antibody can have a variety of structural configurations. For example, a bispecific antibody may consist of two Fc fragments and two binding portions fused thereto, respectively (similar to a natural antibody, except that the two arms bind to different antigen targets or epitopes). Antigen-binding portions can be a single-chain fragment variable (scFv) or Fab fragments. When two non-overlapping epitopes of the same antigen are given, two different binding portions of a bispecific antibody each binds to the N-terminus of one Fc fragment. The configuration of the antigen-binding portions of the two arms can have four combinations, i.e., scFv+Fab fragment, Fab fragment+scFv, scFv+scFv, and Fab fragment+Fab fragment. Fc fragments can contain mutations that can ensure heavy chain heteromerization. The KIH (knob-in-hole) technique is one strategy to address heavy chain heteromerization. Generally, the KIH technique refers to formation of a structure that facilitates the pairing of heterogeneous hemibodies by engineering the amino acid sequence of a CH3 region. This can maintain the structure of a normal antibody as much as possible while forming a bispecific antibody. For guidance of the KIH technique, see, for example, "An efficient route to human bispecific IgG", A. Margaret Merchant et al., Nature Biotechnology, Volume 16, 1998, which is incorporated herein by reference in its entirety. Furthermore, a bispecific antibody can be configured in a way that an antibody that binds to a first epitope of an antigen (e.g., in the form of a natural antibody) has an antigen-binding portion that can bind to a second epitope of the antigen extending from the C-terminus of the CH₃ region (e.g., via a flexible linker).

As used herein, the term "binding portion" or "binding fragment" can be used interchangeably, and refers to a portion or region of an intact antibody molecule responsible for binding to an antigen. An antigen binding domain can comprise a heavy chain variable region (VH), a light chain variable region (VL), or both. Each of VH and VL typically contains three complementarity determining regions, *i.e.,* CDR1, CDR2, and CDR3.

It is well known to those skilled in the art that complementarity determining regions (CDRs, usually including CDR1, CDR2 and CDR3) are the regions of a variable region that have mostly impact on the affinity and specificity of an antibody. The CDR sequences of VH or VL have two common definitions, *i.e.,* the Kabat definition and the Chothia definition (see, e.g., Kabat, "Sequences of Proteins of Immunological Interest", National Institutes of Health, Bethesda, Md. (1991); A1-Lazikani et al., J. Mol. Biol. 273: 927-948 (1997); and Martin et al., Proc. Natl. Acad. Sci. USA 86: 9268-9272 (1989)). For the variable region sequences of a given antibody, the sequences of CDR regions in VH and VL can be determined according to the Kabat definition or the Chothia definition. In an embodiment of the present application, CDR sequences are defined according to Kabat.

For the variable region sequences of a given antibody, the sequences of CDR regions in the variable region sequences can be determined in a variety of ways, for example, using online software Abysis (http://www.abysis.org/).

For conventional antibodies, examples of an antigen-binding fragment include, but are not limited to, (1) an Fab fragment, which can be a monovalent fragment having a VL-CL chain and a VH-CH1 chain; (2) an F(ab')₂ fragment, which can be a divalent fragment having two Fab' fragments linked by a disulfide bridge of the hinge region (*i.e.,* a dimer of Fab'); (3) an Fv fragment having VL and VH domains in a single arm of an antibody; (4) a single-chain Fv (scFv), which can be a single polypeptide chain consisting of a VH domain and a VL domain via a polypeptide linker; (5) (scFv)₂, which can comprise two VH domains linked by a peptide linker and two VL domains that are combined with the two VH domains via a disulfide bridge; and (6) a VHH antibody.

In a bispecific antibody construct, a "binding portion" includes, but is not limited to, a Fab fragment or a single-chain fragment variable (scFv).

As used herein, the term "a single chain fragment variable (scFv)" refers to an antibody of a single chain structure generally constructed using genetic engineering techniques, comprising a polypeptide chain comprising a heavy chain variable region (VH) and a light chain variable region (VL). A flexible linker is typically designed between the heavy chain variable region and the light chain variable region so that the heavy chain variable region and the light chain variable region can be folded into the correct conformation for binding to an antigen.

As used herein, the term "Fab (fragment antigen-binding) fragment", "Fab portion", or the like refers to an antibody fragment that is produced after treatment of an intact antibody with papain and is capable of binding to an antigen, including the intact light chain (VL-CL), the heavy chain variable region, and the CH1 fragment (VH-CH1).

As used herein, the terms "first heavy chain constant region" and "second heavy chain constant region" both refer to "Fc fragment", "Fc domain" and "Fc portion" or similar terms, and refer to a part of the antibody heavy chain constant region, including hinge region, CH2 fragment and CH3 fragment of the heavy chain constant region, and are determined with reference to EU numbering of human IgG1 antibody.

As used herein, the term "monoclonal antibody" refers to an antibody from a substantially homogeneous antibody population, which means that the antibodies constituting the population are the same except for naturally occurring mutations which may occur in a small number of individual antibodies. Monoclonal antibodies described herein particularly include "chimeric" antibodies in which a portion of the heavy chain and/or the light chain is identical or homologous to a corresponding sequence in an antibody derived from a particular species or belonging to a particular antibody type or subtype, while the remaining portion of the heavy chain and/or the light chain is identical or homologous to a corresponding sequence in an antibody derived from another species or belonging to another antibody type or subtype; and also include fragments of such antibodies as long as they are capable of exhibiting the desired biological activity (see, U.S. Pat. No. 4,816,567; and Morrison et al., Proc. Natl. Acad. Sci. USA 81: 6851-6855 (1984)).

As used herein, the term "specific binding" refers to a non-random binding reaction between two molecules, e.g., binding of an antibody to an antigen epitope.

In a first aspect, there is provided in the present application a bispecific antibody comprising a first binding fragment and a second binding fragment that bind to non-overlpapping epitopes of human thymic stromal lymphopoietin (TSLP).

In some embodiments of the first aspect, the bispecific antibody is capable of blocking the binding of human TSLP to human TSLP receptor (TSLPR), and the bispecific antibody is capable of blocking the binding of human IL-7 receptor alpha chain (IL-7Rα) to human TSLP: TSLPR complex.

In some embodiments of the first aspect, the epitope of human TSLP bound by one of the first binding fragment and the second binding fragment partially overlaps with the epitope of human TSLP bound by IL-7Rα.

In some embodiments of the first aspect, the first binding fragment comprises HCDR1 as set forth in SEQ ID NO:29, HCDR2 as set forth in SEQ ID NO:30, HCDR3 as set forth in SEQ ID NO:31, LCDR1 as set forth in SEQ ID NO:32, LCDR2 as set forth in SEQ ID NO:33, and LCDR3 as set forth in SEQ ID NO:34; wherein the amino acid sequences of HCDRs and LCDRs are defined according to Kabat.

In some embodiments of the first aspect, the second binding fragment comprises HCDR1 as set forth in SEQ ID NO:35, HCDR2 as set forth in SEQ ID NO:36, HCDR3 as set forth in SEQ ID NO:37, LCDR1 as set forth in SEQ ID NO:32, LCDR2 as set forth in SEQ ID NO:33, and LCDR3 as set forth in SEQ ID NO:34; wherein the amino acid sequences of HCDRs and LCDRs are defined according to Kabat.

In some embodiments of the first aspect, the amino acid sequence of the heavy chain variable region of the first binding fragment is set forth in SEQ ID NO: 24, and the amino acid sequence of the light chain variable region of the first binding fragment is set forth in SEQ ID NO: 21; or
the amino acid sequence of the heavy chain variable region of the first binding fragment is set forth in SEQ ID NO: 24, and the amino acid sequence of the light chain variable region of the first binding fragment is set forth in SEQ ID NO: 28.

In some embodiments of the first aspect, the amino acid sequence of the heavy chain variable region of the second binding fragment is set forth in SEQ ID NO: 20, and the amino acid sequence of the light chain variable region of the second binding fragment is set forth in SEQ ID NO: 21; or
the amino acid sequence of the heavy chain variable region of the second binding fragment is set forth in SEQ ID NO: 20, and the amino acid sequence of the light chain variable region of the second binding fragment is set forth in SEQ ID NO: 28.

In some embodiments of the first aspect, the bispecific antibody is an IgG1 antibody comprising a first heavy chain constant region and a second heavy chain constant region, wherein the amino acids at positions 354 and 366 of the first heavy chain constant region are C and W, respectively, and the amino acids at positions 349, 366, 368 and 407 of the second heavy chain constant region are C, S, A and V, respectively; and the amino acid positions of antibody constant regions are determined according to EU numbering.

In some embodiments of the first aspect, the bispecific antibody is an IgG1 antibody comprising a first heavy chain constant region and a second heavy chain constant region, wherein the amino acids at positions 234, 235 and 331 of the first heavy chain constant region and the second heavy chain constant region are F, E and S, respectively; and the amino acid positions of antibody constant regions are determined according to EU numbering.

In some embodiments of the first aspect, the forms of the first binding fragment and the second binding fragment are independently selected from a single-chain fragment variable (scFv) or a Fab fragment.

In some embodiments of the first aspect, both of the first binding fragment and the second binding fragment are Fab fragments.

In some embodiments of the first aspect, the first binding fragment and the second binding fragment have the same light chain variable region.

In some embodiments of the first aspect, the first binding fragment and the second binding fragment have the same light chain.

In some embodiments of the first aspect, the bispecific antibody comprises the heavy chain as set forth in SEQ ID NO:38 and the light chain as set forth in one of SEQ ID NOs: 39 and 40.

In some embodiments of the first aspect, the bispecific antibody comprises the heavy chain as set forth in SEQ ID NO:41 and the light chain as set forth in one of SEQ ID NOs: 39 and 40.

In some embodiments of the first aspect, the amino acid sequence of the heavy chain variable region of the first binding fragment differs from the amino acid sequence as set forth in SEQ ID NO:24 by about 1, 2, 3, 4, 5, 6, 7, 8, 9, or 10 amino acid substitutions, deletions, and/or additions.

In some embodiments of the first aspect, the amino acid sequence of the light chain variable region of the first binding fragment differs from the amino acid sequence as set forth in SEQ ID NO:21 or 28 by about 1, 2, 3, 4, 5, 6, 7, 8, 9, or 10 amino acid substitutions, deletions, and/or additions.

In some embodiments of the first aspect, the amino acid sequence of the heavy chain variable region of the first binding fragment has at least 90%, 91%, 92%, 93%, 94%, 95%, 96%, 97%, 98%, 99% or more homology to SEQ ID NO:24.

In some embodiments of the first aspect, the amino acid sequence of the light chain variable region of the first binding fragment has at least 90%, 91%, 92%, 93%, 94%, 95%, 96%, 97%, 98%, 99% or more homology to SEQ ID NO:21 or 28.

In some embodiments of the first aspect, the C-terminal or N-terminal region of the amino acid sequence as set forth in SEQ ID NO:24 can also be truncated by about 1, 2, 3, 4, 5, 6, 7, 8, 9, 10, 11, 12, 13, 14, 15, 20, 25 or more amino acids while still retaining the function similar to that of the heavy chain variable region of the first binding fragment.

In some embodiments of the first aspect, 1, 2, 3, 4, 5, 6, 7, 8, 9, 10, 11, 12, 13, 14, 15, 20, 25 or more amino acids can also be added to the C-terminal or N-terminal region of the amino acid sequence as set forth in SEQ ID NO:24, and the resulting amino acid sequences still retain the function similar to that of the heavy chain variable region of the first binding fragment.

In some embodiments of the first aspect, 1, 2, 3, 4, 5, 6, 7, 8, 9, 10, 11, 12, 13, 14, 15, 20, 25 or more amino acids can also be added or deleted at a region other than the C-terminus or the N-terminus of the amino acid sequence as set forth in SEQ ID NO:24, provided that the altered amino acid sequences substantially retain the function similar to that of the heavy chain variable region of the first binding fragment.

In some embodiments of the first aspect, the C-terminal or N-terminal region of the amino acid sequence as set forth in SEQ ID NO:21 or 28 can also be truncated by about 1, 2, 3, 4, 5, 6, 7, 8, 9, 10, 11, 12, 13, 14, 15, 20, 25 or more amino acids while still retaining the function similar to that of the light chain variable region of the first binding fragment.

In some embodiments of the first aspect, 1, 2, 3, 4, 5, 6, 7, 8, 9, 10, 11, 12, 13, 14, 15, 20, 25 or more amino acids can also be added to the C-terminal or N-terminal region of the amino acid sequence as set forth in SEQ ID NO:21 or 28, and the resulting amino acid sequences still retain the function similar to that of the light chain variable region of the first binding fragment.

In some embodiments of the first aspect, 1, 2, 3, 4, 5, 6, 7, 8, 9, 10, 11, 12, 13, 14, 15, 20, 25 or more amino acids can also be added or deleted at a region other than the C-terminus or the N-terminus of the amino acid sequence as set forth in SEQ ID NO:21 or 28, provided that the altered amino acid sequences substantially retain the function similar to that of the light chain variable region of the first binding fragment.

In some embodiments of the first aspect, the amino acid sequence of the heavy chain variable region of the second binding fragment differs from the amino acid sequence as set forth in SEQ ID NO:20 by about 1, 2, 3, 4, 5, 6, 7, 8, 9, or 10 amino acid substitutions, deletions, and/or additions.

In some embodiments of the first aspect, the amino acid sequence of the light chain variable region of the second binding fragment differs from the amino acid sequence as set forth in SEQ ID NO:21 or 28 by about 1, 2, 3, 4, 5, 6, 7, 8, 9, or 10 amino acid substitutions, deletions, and/or additions.

In some embodiments of the first aspect, the amino acid sequence of the heavy chain variable region of the second binding fragment has at least 90%, 91%, 92%, 93%, 94%, 95%, 96%, 97%, 98%, 99% or more homology to SEQ ID NO:20.

In some embodiments of the first aspect, the amino acid sequence of the light chain variable region of the second binding fragment has at least 90%, 91%, 92%, 93%, 94%, 95%, 96%, 97%, 98%, 99% or more homology to SEQ ID NO:21 or 28.

In some embodiments of the first aspect, the C-terminal or N-terminal region of the amino acid sequence as set forth in SEQ ID NO:20 can also be truncated by about 1, 2, 3, 4, 5, 6, 7, 8, 9, 10, 11, 12, 13, 14, 15, 20, 25 or more amino acids while still retaining the function similar to that of the heavy chain variable region of the second binding fragment.

In some embodiments of the first aspect, 1, 2, 3, 4, 5, 6, 7, 8, 9, 10, 11, 12, 13, 14, 15, 20, 25 or more amino acids can also be added to the C-terminal or N-terminal region of the amino acid sequence as set forth in SEQ ID NO: 20, and the resulting amino acid sequences still retain the function similar to that of the heavy chain variable region of the second binding fragment.

In some embodiments of the first aspect, 1, 2, 3, 4, 5, 6, 7, 8, 9, 10, 11, 12, 13, 14, 15, 20, 25 or more amino acids can also be added or deleted at a region other than the C-terminus or the N-terminus of the amino acid sequence as set forth in SEQ ID NO: 20, provided that the altered amino acid sequences substantially retain the function similar to that of the heavy chain variable region of the second binding fragment.

In some embodiments of the first aspect, the C-terminal or N-terminal region of the amino acid sequence as set forth in SEQ ID NO: 21 or 28 can also be truncated by about 1, 2, 3, 4, 5, 6, 7, 8, 9, 10, 11, 12, 13, 14, 15, 20, 25 or more amino acids while still retaining the function similar to that of the light chain variable region of the second binding fragment.

In some embodiments of the first aspect, 1, 2, 3, 4, 5, 6, 7, 8, 9, 10, 11, 12, 13, 14, 15, 20, 25 or more amino acids can also be added to the C-terminal or N-terminal region of the amino acid sequence as set forth in SEQ ID NO:21 or 28, and the resulting amino acid sequences still retain the function similar to that of the light chain variable region of the second binding fragment.

In some embodiments of the first aspect, 1, 2, 3, 4, 5, 6, 7, 8, 9, 10, 11, 12, 13, 14, 15, 20, 25 or more amino acids can also be added or deleted at a region other than the C-terminus or the N-terminus of the amino acid sequence as set forth in SEQ ID NO:21 or 28, provided that the altered amino acid sequences substantially retain the function of the light chain variable region of the second binding fragment.

In a second aspect, there is provided in the present application a monoclonal antibody that binds to human TSLP, comprising HCDR1 as set forth in SEQ ID NO:29, HCDR2 as set forth in SEQ ID NO:30, HCDR3 as set forth in SEQ ID NO:31, LCDR1 as set forth in SEQ ID NO:32, LCDR2 as set forth in SEQ ID NO:33, and LCDR3 as set forth in SEQ ID NO:34; wherein the amino acid sequences of HCDR and LCDR are defined according to Kabat.

In some embodiments of the second aspect, the amino acid sequence of the heavy chain variable region of the monoclonal antibody is set forth in SEQ ID NO:24 and the amino acid sequence of the light chain variable region is set forth in SEQ ID NO:21; or the amino acid sequence of the heavy chain variable region of the monoclonal antibody is set forth in SEQ ID NO:24, and the amino acid sequence of the light chain variable region is set forth in SEQ ID NO:28.

In some embodiments of the second aspect, the amino acid sequence of the heavy chain variable region of the monoclonal antibody differs from the amino acid sequence as set forth in SEQ ID NO:24 by about 1, 2, 3, 4, 5, 6, 7, 8, 9, or 10 amino acid substitutions, deletions, and/or additions.

In some embodiments of the second aspect, the amino acid sequence of the light chain variable region of the monoclonal antibody differs from the amino acid sequence as set forth in SEQ ID NO:21 or 28 by about 1, 2, 3, 4, 5, 6, 7, 8, 9, or 10 amino acid substitutions, deletions, and/or additions.

In some embodiments of the second aspect, the amino acid sequence of the heavy chain variable region of the monoclonal antibody has at least 90%, 91%, 92%, 93%, 94%, 95%, 96%, 97%, 98%, 99% or more homology to SEQ ID NO:24.

In some embodiments of the second aspect, the amino acid sequence of the light chain variable region of the monoclonal antibody has at least 90%, 91%, 92%, 93%, 94%, 95%, 96%, 97%, 98%, 99% or more homology to SEQ ID NO:21 or 28.

In some embodiments of the second aspect, the C-terminal or N-terminal region of the amino acid sequence as set forth in SEQ ID NO:24 can also be truncated by about 1, 2, 3, 4, 5, 6, 7, 8, 9, 10, 11, 12, 13, 14, 15, 20, 25 or more amino acids while still retaining the function similar to that of the heavy chain variable region of the monoclonal antibody.

In some embodiments of the second aspect, 1, 2, 3, 4, 5, 6, 7, 8, 9, 10, 11, 12, 13, 14, 15, 20, 25 or more amino acids can also be added to the C-terminal or N-terminal region of the amino acid sequence as set forth in SEQ ID NO: 24, and the resulting amino acid sequences still retain the function similar to that of the heavy chain variable region of the monoclonal antibody.

In some embodiments of the second aspect, 1, 2, 3, 4, 5, 6, 7, 8, 9, 10, 11, 12, 13, 14, 15, 20, 25 or more amino acids can also be added or deleted at a region other than the C-terminus or the N-terminus of the amino acid sequence as set forth in SEQ ID NO: 24, provided that the altered amino acid sequences substantially retain the function similar to that of the heavy chain variable region of the monoclonal antibody.

In some embodiments of the second aspect, the C-terminal or N-terminal region of the amino acid sequence as set forth in SEQ ID NO:21 or 28 can also be truncated by about 1, 2, 3, 4, 5, 6, 7, 8, 9, 10, 11, 12, 13, 14, 15, 20, 25 or more amino acids while still retaining the function similar to that of the light chain variable region of the monoclonal antibody.

In some embodiments of the second aspect, 1, 2, 3, 4, 5, 6, 7, 8, 9, 10, 11, 12, 13, 14, 15, 20, 25 or more amino acids can also be added to the C-terminal or N-terminal region of the amino acid sequence as set forth in SEQ ID NO:21 or 28, and the resulting amino acid sequences still retain the function similar to that of the light chain variable region of the monoclonal antibody.

In some embodiments of the second aspect, 1, 2, 3, 4, 5, 6, 7, 8, 9, 10, 11, 12, 13, 14, 15, 20, 25 or more amino acids can also be added or deleted at a region other than the C-terminus or the N-terminus of the amino acid sequence as set forth in SEQ ID NO:21 or 28, provided that the altered amino acid sequences substantially retain the function similar to that of the light chain variable region of the monoclonal antibody.

In a third aspect, there is provided in the present application a pharmaceutical composition comprising the bispecific antibody of the first aspect, or the monoclonal antibody of the second aspect, and a pharmaceutically acceptable excipient, diluent, or carrier.

In some embodiments of the third aspect, the pharmaceutical composition is used for preventing or treating a TSLP-mediated disease.

In some embodiments of the third aspect, the TSLP-mediated disease is selected from the group consisting of asthma, scleroderma, systemic lupus erythematosus, rheumatoid arthritis, Churg-Strauss syndrome, Wegener's granulomatosis, allergic pneumonia, atopic dermatitis, rhinitis, Crohn's disease, psoriatic arthritis, chronic nephritis, Sjogren's syndrome, and multiple sclerosis.

In some embodiments of the third aspect, the pharmaceutical composition can further comprise one or more of a lubricant, such as talc, magnesium stearate, and mineral oil; a wetting agent; an emulsifier; a suspending agent; a preservative such as benzoic acid, sorbic acid and calcium propionate; a sweetening agent and/or a flavoring agent.

In some embodiments of the third aspect, the pharmaceutical composition herein can be formulated as a tablet, a pill, a powder, a lozenge, an elixir, a suspension, an emulsion, a solution, a syrup, a suppository, or a capsule.

In some embodiments of the third aspect, the pharmaceutical composition of the present application can be delivered using any physiologically acceptable administration route including, but not limited to, oral administration, parenteral administration, nasal administration, rectal administration, intraperitoneal administration, intravascular injection, subcutaneous administration, transdermal administration, or inhalation administration.

In some embodiments of the third aspect, a pharmaceutical composition for therapeutic use can be formulated for storage in the form of a lyophilized formulation or an aqueous solution by mixing an agent with desired purity with a pharmaceutically acceptable carrier or excipient where appropriate.

In a fourth aspect, there is provided in the present application use of the bispecific antibody of the first aspect, the monoclonal antibody of the second aspect, or the pharmaceutical composition of the third aspect in the manufacture of a medicament for the prevention or treatment of a TSLP-mediated disease.

In some embodiments of the fourth aspect, the TSLP-mediated disease is selected from the group consisting of asthma, scleroderma, systemic lupus erythematosus, rheumatoid arthritis, Churg-Strauss syndrome, Wegener's granulomatosis, allergic pneumonia, atopic dermatitis, rhinitis, Crohn's disease, psoriatic arthritis, chronic nephritis, Sjogren's syndrome, and multiple sclerosis.

In a fifth aspect, there is provided in the present application a method of preventing or treating a TSLP-mediated disease, comprising administering to a subject in need thereof the bispecific antibody of the first aspect, the monoclonal antibody of the second aspect, or the pharmaceutical composition of the third aspect.

In some embodiments of the fifth aspect, the TSLP-mediated disease is selected from the group consisting of asthma, scleroderma, systemic lupus erythematosus, rheumatoid arthritis, Churg-Strauss syndrome, Wegener's granulomatosis, allergic pneumonia, atopic dermatitis, rhinitis, Crohn's disease, psoriatic arthritis, chronic nephritis, Sjogren's syndrome, and multiple sclerosis.

In other aspects, there is provided in the present application a nucleic acid molecule encoding the bispecific antibody of the first aspect, or the monoclonal antibody of the second aspect. In some embodiments, the nucleic acid molecule is operably linked to a regulatory sequence that can be recognized by a host cell transformed with the vector.

There is also provided in the present application a vector comprising an isolated nucleic acid molecule encoding the bispecific antibody of the first aspect, or the monoclonal antibody of the second aspect, and a host cell comprising the nucleic acid molecule or the vector. In other aspects, there is provided in the present application a method of producing the bispecific antibody of the first aspect, or the monoclonal antibody of the second aspect. In some embodiments, the method of producing the bispecific antibody of the first aspect, or the monoclonal antibody of the second aspect comprises culturing a host cell so as to facilitate expression of the nucleic acid molecule. In some embodiments, the method of producing the bispecific antibody of the first aspect, or the monoclonal antibody of the second aspect further comprises recovering the bispecific antibody or monoclonal antibody from the host cell culture medium.

It is to be understood that the foregoing detailed description is intended only to enable those skilled in the art to have better understanding of the present application and is not intended to cause limitations in any way. Various modifications and variations can be made to the described embodiments by those skilled in the art.

The following Examples are for purposes of illustration only and are not intended to limit the scope of the present application.

### Examples

### Example 1: Preparation of recombinant proteins

A number of different recombinant proteins are needed in the preparation of anti-TSLP antibodies, including the long version of the extracellular domain of human TSLP (hTSLP1-ECD, SEQ ID NO:1), the short version of the extracellular domain of human TSLP (hTSLP2-ECD, SEQ ID NO:2), the extracellular domain of mouse TSLP (mTSLP-ECD, SEQ ID NO:3), and the extracellular domain of cynomolgus monkey TSLP (mfTSLP1-ECD, SEQ ID NO:4). These proteins have a large number of post-translational modifications (e.g., glycosylation or disulfide bonds, etc), and therefore, the use of the mammal cell expression system would be more favorable for maintaining the structure and function of the recombinant proteins. In addition, in order to avoid the effect of the furin recognition sites in hTSLP1, hTSLP2 and mfTSLP1 on the activity of the TSLP protein, hTSLP1 mutant (hTSLP1-m, SEQ ID NO:5), hTSLP2 mutant (hTSLP2-m, SEQ ID NO:6) and mfTSLP1 mutant (mf ISLP1-m, SEQ ID NO:7) with deletion of the furin recognition sites were respectively constructed. Meanwhile, the addition of the His tag (His, SEQ ID NO:8) or the Fc fragment of the human antibody IgG1 (hFc, SEQ ID NO:9) or the Fc fragment of the mouse antibody IgG2a (mFc, SEQ ID NO:10) to the C-terminus of these recombinant proteins will be more favorable for the purification of the recombinant proteins and the identification of the monoclonal antibody functions. The antibody heavy chain constant region can be human IgG1 subtype (SEQ ID NO:11), human IgG2 subtype (SEQ ID NO:12), human IgG4 subtype (SEQ ID NO:13), or mouse IgG1 subtype (SEQ ID NO:14), or mouse IgG2a subtype (SEQ ID NO:15), and the light chain constant region can be human kappa subtype (SEQ ID NO:16), human lambda subtype (SEQ ID NO:17), mouse kappa subtype (SEQ ID NO:18), or mouse lambda subtype (SEQ ID NO:19).

According to the amino acid sequences of various recombinant proteins of interest in the Uniprot database, genes (including His tags or hFc or mFc encoding genes) encoding the above various recombinant proteins were designed and synthesized. Various synthesized genes encoding the recombinant proteins were cloned into suitable eukaryotic expression vectors (such as pcDNA3.1, Invitrogen Inc., etc) using conventional molecular biology techniques, and the prepared recombinant antibody expression plasmids were then transfected into HEK293 cells (such as HEK293F, Invitrogen, Inc.) using liposomes (such as 293fectin, Invitrogen, Inc., etc) or other cationically transfected reagents (such as PEI, etc). The cells were cultured in suspension in serum-free mediums for 3-4 days. The culture supernatant was then harvested by centrifugation.

The recombinant protein expressed by fusion with His-tag was subjected to one-step purification of the recombinant protein in the culture supernatant using a metal chelate affinity chromatography column (such as HisTrap FF, GE, Inc, etc). The recombinant protein expressed by the fusion of hFc and mFc was further purified by a ProteinA/G affinity chromatography column (such as Mabselect SURE, GE, Inc., etc). The recombinant protein preservation buffer was then replaced with PBS (pH 7.0) or other suitable buffers by using a desalting column (such as Hitrap desaulting, GE, Inc., etc). If necessary, the antibody sample can be sterilized by filtration and then stored in aliquots at -20°C.

### Example 2: Construction and screening of a fixed light chain-based TSLP mouse immune library

In order to construct a TSLP bispecific antibody based on a common light chain, a light chain variable region of an anti-TSLP monoclonal antibody against a specific epitope was selected in conjunction with a mouse heavy chain variable region which had been matured with a TSLP antigen *in vivo,* and a single-chain fragment variable (scFv) library was constructed by using conventional molecular biological means for screening anti-TSLP monoclonal antibodies against different epitopes.

Splenocytes were collected from 6-8 week old BALB/c mice after immunisation using hTSLP1-m-His recombinant protein as immunogen. Mouse spleen lymphocytes were isolated by using a mouse lymphocyte isolation solution (Dakewe Biotech Co., Ltd., CAT#DKW33-R0100). Total RNA was extracted from the isolated lymphocytes by using a cellular total RNA extraction kit (Tiangen Biotech (Beijing) Co., Ltd., CAT#DP430). Using the extracted total RNA as a template, the heavy chain variable region of the antibody was synthesized using a first chain cDNA synthesis kit (Thermo scientific, CAT#K1621). The light chain variable region of the monoclonal antibody H1A10+L3E8-M1 that recognized the specific epitope of hTSLP1 was obtained by PCR amplification using conventional molecular biological means (see the humanized antibody H1A10+L3E8-M1 in Chinese Patent Application No. 202010080450.1, with the amino acid sequence of the heavy chain variable region as set forth in SEQ ID NO:20, and the amino acid sequence of the light chain variable region as set forth in SEQ ID NO:21) and the mouse heavy chain variable region was obtained by immunizing mice with hTSLP1-m-His recombinant antigen. Then, the single-chain fragment variables (scFv) was constructed by overlapping extension PCR technology. The genes encoding the prepared mouse single chain fragment variables were cloned into the vector pADSCFV-S (see Example 1 of the Chinese Patent Application No. 201510097117.0 for detailed experimental protocols) to construct a scFv library. The antibody library has a capacity of 8.3E+07, and an accuracy of 65%.

Referring to the reference (Chinese Patent Application No. 201510097117.0), the constructed fixed light chain immune library of mouse was screened by a solid phase screening strategy (as for the experimental protocol, see, Phage Display: A Practical Approach, Tim Clackson (USA) and Henry B. Lowman (USA) (ed.), translated by Lan Ma et al., Chemical Industry Press, May 2008) using the recombinant hTSLP1-m-his prepared in Example 1 as the antigen. Three rounds of screening were carried out by means of binding, elution, neutralization, infection and amplification. Finally, one single chain fragment variable S17C7+L3E8-M1 specifically binding to human TSLP was obtained.

### Example 3: Identification of anti-hTSLP mouse monoclonal antibody

The nucleic acid molecules encoding the light variable region and the heavy chain variable region of S17C7+L3E8-M1 were respectively cloned into eukaryotic expression vectors by using conventional molecular biological methods so as to prepare and express the whole antibody. Meanwhile, the humanized anti-TSLP monoclonal antibody Tezepelumab was prepared according to U.S. Pat. No. US9284372B2 as a positive control (the amino acid sequence of the heavy chain variable region is set forth in SEQ ID NO:22; and the amino acid sequence of the light chain variable region is set forth in SEQ ID NO:23).

### 3.1 Affinity analysis of the recombinant anti-TSLP monoclonal antibody S17C7+L3E8-M1

The affinity of the anti-TSLP antibody was determined by the surface plasmon resonance technique using Biacore X100. Reagents and consumables such as an amino-coupling kit (BR-1000-50), a human antibody capture kit (BR-1008-39), a CM5 chip (BR100012) and 10×HBS-EP (pH 7.4, BR100669) were purchased from GE healthcare. The surface of the carboxylated CM5 chip was activated with 1-ethyl-3-(3-dimethylaminopropyl) carbodiimide hydrochlorid (EDC) and N-Hydroxysuccinimide (NHS) according to the instructions in the kit. An anti-human IgG (Fc) antibody (capture antibody) was diluted to 25µg/mL with 10mM sodium acetate (pH5.0), and then injected at a flow rate of 10µL/min to achieve a coupling amount of approximately 10,000 response units (RU). After injection of the capture antibody, 1 M ethanolamine was injected to block unreacted groups. For the kinetics measurement, the anti-TSLP antibody was diluted to 0.5-1µg/mL, and injected at 10µL/min to ensure that approximately 100RU of the antibody was captured by the anti-human Fc antibody. hTSLP1-m-his was then set to a series of concentration gradients (e.g., 0.625nM, 1.25nM, 2.5nM, 5nM, and 10nM) and injected at 25°C from lower to higher concentrations at 30µL/min, with an association time of 120s and a dissociation time of 3,600s. A 3M MgCl₂ solution was injected at 10µL/min for 30s to regenerate the surface of the chip. The association rate (Kₐ) and dissociation rate (K_{d}) were calculated by fitting the binding and dissociation sensing grams with a 1:1 binding model by using Biacore X100 evaluation software version 2.0.1. The dissociation equilibrium constant (K_{D}) was calculated as the ratio K_{d}/Kₐ. The fitting results were shown in Table 1.

**Table 1. Affinity constants for the binding of the recombinant anti-human TSLP monoclonal antibodies to hTSLP**

| Monoclonal antibody | Kₐ | K_{d} | K_{D} |
|---|---|---|---|
| Tezepelumab | 8.633E+6 | 1.278E-5 | 1.48E-12 |
| S17C7+L3E8-M1 | 4.611E+6 | 8.699E-4 | 1.887E-10 |

### 3.2 Inhibition of the binding of TSLP to TSLPR by the recombinant anti-TSLP monoclonal antibody S17C7+L3E8-M1

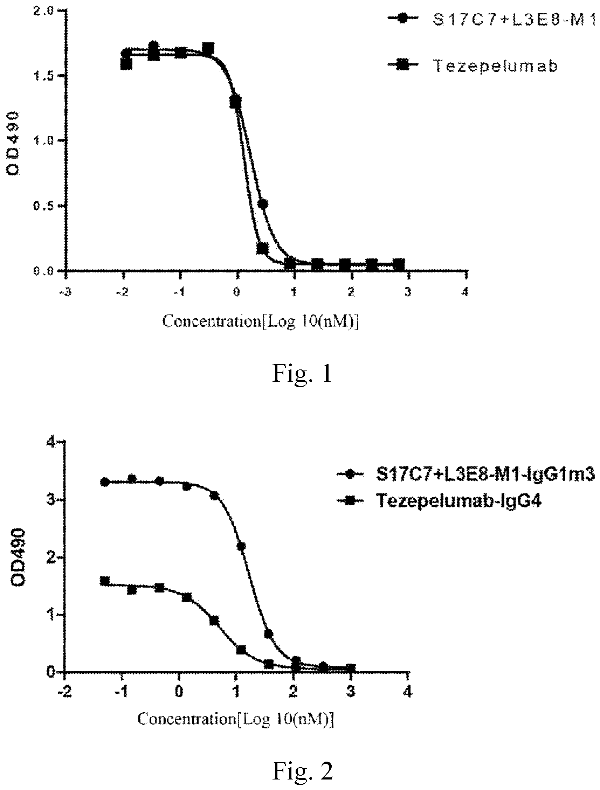

96-well plates were coated with the recombinant antigen TSLPR-Fc (3µg/mL, 100µL/well) overnight at 4°C, and were then blocked with a blocking solution (PBS-0.1% Tween 20-3% milk) at 37°C for 1 hour. Gradient dilutions of the monoclonal antibodies S17C7+L3E8-M1 and Tezepelumab were performed with hTSLP1-m-his at a fixed concentration (0.2µg/mL). The starting concentration of the antibodies was 100µg/mL, and the dilution was carried out at 3-fold for a total of 10 concentration gradients. The antibody dilutions were added to the blocked 96-well ELISA plates at 100 µL/well and the plates were incubated at 37°C for 1 hour. Then, the ELISA plates were washed with PBS-0.1% Tween 20, followed by the addition of 1:5000 diluted HRP-anti-His labeled mouse monoclonal antibody (Beijing Kangwei Century Biotechnology Co., Ltd., CW0285M) at 100 µL/well and the plates were incubated at 37°C for 1 hour. The ELISA plates were washed with PBS-0.1% Tween 20, and an OPD substrate chromogenic solution was added. After incubation for 5-10 minutes, the colour development was terminated with 1M H₂SO₄. The optical density values were measured using a microplate reader at 492nm/630nm dual wavelength. The results from the ELISA assay (FIG. 1) showed that S17C7+L3E8-M1 can completely inhibit the binding of TSLP to TSLPR, and the inhibitory ability of S17C7+L3E8-M1 was comparable to that of Tezepelumab. The IC₅₀ values were shown in Table 2.

**Table 2. IC₅₀ values of anti-human TSLP monoclonal antibodies for inhibiting the binding of TSLP toTSLPR**

| Monoclonal antibody | S17C7+L3E8-M1 | Tezepelumab |
|---|---|---|
| IC₅₀ (nM) | 1.707 | 1.307 |

### 3.3 Comparison of the epitopes of TSLP bound by the recombinant anti-TSLP monoclonal antibodies S17C7+L3E8-M1 and Tezepelumab

96-well plates were coated with recombinant protein hTSLP1-m-his (1µg/mL, 100µL/well) overnight at 4°C, and were then blocked with a blocking solution (PBS-0.1% Tween 20-3% milk) at 37°C for 1 hour. Gradient dilutions of Tezepelumab-IgG4 and S 17C7+L3E8-M1-IgG 1m3 were performed with PBS-2% milk containing recombinant antibody Tezepelumab-mIgG2a at 0.05 µg/mL. The starting concentration of the antibodies was 200µg/mL, and the dilution was carried out at 3-fold for a total of 10 concentration gradients. The antibody dilutions were added to the blocked 96-well ELISA plates at 100 µL/well and the plates were incubated at 37°C for 1 hour. Then, the ELISA plates were washed with PBS-0.1% Tween 20, followed by the addition of 1:5000 diluted HRP-goat-anti-mouse IgG (Beijing Zhongshan Jinqiao Biotechnology Co., Ltd., ZB2305) at 100 µL/well and the plates were incubated at 37°C for 1 hour. The ELISA plates were washed with PBS-0.1% Tween 20, and an OPD substrate chromogenic solution was added. After incubation for 5-10 minutes, the colour development was terminated with 1M H₂SO₄. The optical density values were measured using a microplate reader at 492nm/630nm dual wavelength. Data were analyzed and graphed using GraphPad Prism 6. The results from the ELISA assay (FIG. 2) showed that S 17C7+L3E8-M1-IgG 1m3 can completely inhibit the binding of Tezepelumab-mIgG2a to TSLP. The IC₅₀ values were shown in Table 3. The IC₅₀ value of S 17C7+L3E8-M1-IgG 1m3 for inhibiting the binding of Tezepelumab-mIgG2a to TSLP was 2.2-fold higher than that of Tezepelumab-IgG4 for inhibiting the binding of Tezepelumab-mIgG2a to TSLP, which may be related to the lower affinity of the binding of S 17C7+L3E8-M1-IgG 1m3 to TSLP than that of the binding of Tezepelumab-IgG4 to TSLP (see Table 1).

**Table 3. IC₅₀ values of anti-human TSLP monoclonal antibodies for inhibiting the binding of Tezepelumab-mIgG2a to TSLP**

| Monoclonal antibody | S17C7+L3E8-M1-IgG1m3 | Tezepelumab-IgG4 |
|---|---|---|
| IC₅₀ (nM) | 3.223 | 1.467 |

### 3.4 Inhibition of the proliferation-promoting effect of TSLP on BAF3-TSLPR/IL-7Rα cells by the recombinant anti-human TSLP monoclonal antibody S17C7+L3E8-M1

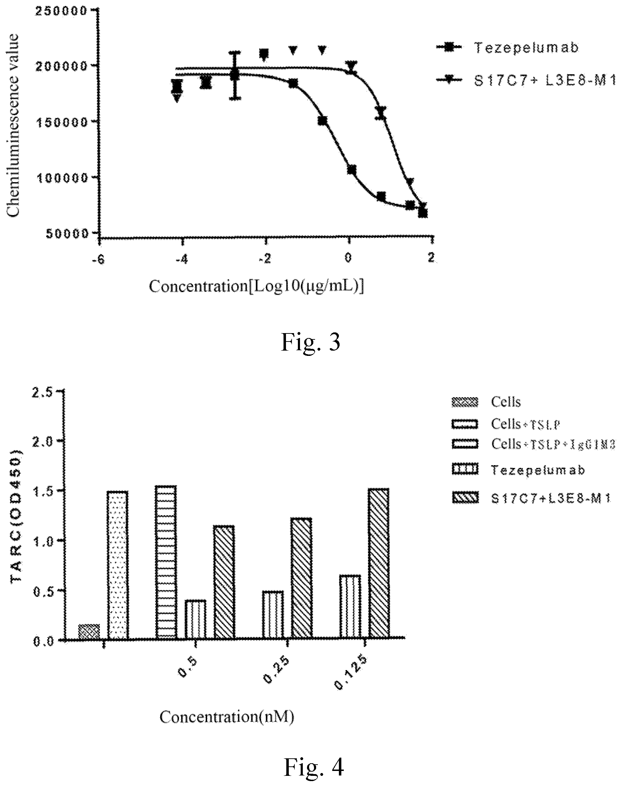

BAF3-TSLPR/IL-7Rα was a stable transgenic cell strain with high expression of the TSLP receptor and its proliferation was dependent on mIL-3. In the absence of mIL-3, TSLP can effectively promote the proliferation of BA/F3-TSLPR/IL-7Rα cells. This method can be used to evaluate anti-human TSLP monoclonal antibody to inhibit the proliferation-promoting effect of TSLP on BAF3-TSLPR/IL-7Rα cells. In the experiment, BAF3-TSLPR/IL-7Rα cells were pre-cultured for two days using complete medium without mIL-3. On the day of the experiment, cells were centrifugated and resuspended to a density of 1×10⁶ cells/mL, and added to 96-well flat bottom cell culture plates at 50µL/wells. The dilutions of the recombinant anti-human TSLP monoclonal antibody S17C7+L3E8-M1 and the positive control antibody (i.e., Tezepelumab) were both started at a final concentration of 60µg/mL, with a second concentration of 30µg/mL, followed by a 5-fold gradient of each of 8 concentration gradients. The diluted anti-human TSLP monoclonal antibody was premixed with an equal volume of hTSLP-m-his at a final concentration of 0.4 ng/mL for 30 minutes, and then the mixture was mixed with BAF3-TSLPR/IL-7Rα cells and incubated in a total volume of 150µL/wells. After 48 hours, the cell proliferation was determined by a multifunctional microplate reader (SpectraMax^{®}I3X) using the Cell Titer-Glo^{®} Luminescent Cell Viability Assay Kit (Promega, Cat#G7570). The results of chemiluminescence readings were fitted and analyzed. The results (FIG.3) showed that both of the anti-human monoclonal antibody S17C7+L3E8-M1 and the positive control antibody (i.e., Tezepelumab) were effective in inhibiting the proliferation-promoting effect of TSLP on BAF3-TSLPR/IL-7Rα cells.

### 3.5 Effect of the recombinant anti-human TSLP monoclonal antibody S17C7+L3E8-M1 on inhibiting the secretion of TARC by PBMCs stimulated by TSLP

Blood was collected from normal volunteers (50 mL each), where the collected blood was provided by the inventors and colleagues thereof as volunteers, and all of them had signed informed consent. The inclusion criteria for volunteers were:
1. older than 18 years;
2. no HIV and HBV infections;
3. normal blood routine tests;
4. non-pregnant or lactating women.

Human peripheral blood mononuclear cells (PBMCs) were isolated from healthy human peripheral blood using Ficoll density gradient centrifugation.

The binding of TSLP to TSLPR on dendritic cells can cause activation of the dendritic cells, which exhibits the secretion of chemokines (e.g., TARC, also known as CCL17). The inhibitory effect of recombinant anti-TSLP antibodies on TSLP can be effectively evaluated by detecting TARC. PBMCs were resuspended in R1640 medium containing 10% (inactivated) serum to a density of 2×10⁷ cells/mL and were added to 96-well flat bottom cell culture plates (50µL/well). The dilutions of the recombinant anti-human TSLP monoclonal antibody S17C7+L3E8-M1 and the positive control antibody (i.e., Tezepelumab) were both started at a final concentration of 0.5 nM, with a 2-fold gradient for a total of 3 working concentrations. The diluted recombinant anti-human TSLP monoclonal antibody was premixed with an equal volume of hTSLP-m-his at a final concentration of 1ng/mL for 30 minutes, and then the mixture was added to the cells for mixing, with a total volume of 150µL/well. No TSLP control (cells), no antibody control (cells +TSLP), and IgG1m3 isotype antibody control (cells +TSLP+IgG1m3) were set. The mixture was incubated at 37°C, 5% CO₂ for 24 hours. The supernatant was then taken and the TARC was detected by using the humanized antibody TARC matched ELISA antibody pair set (Sino Biological Inc., Cat# SEK10233) based on an ELISA assay. The chromogenic solution was a TMB high-sensitivity substrate solution (Biolegend, Cat# 421501). The optical density value of OD450 was determined using a microplate reader (Biotek, Cat# ELX800). The readings were analyzed. The results (FIG. 4) showed that both of the recombinant anti-human TSLP monoclonal antibody S17C7+L3E8-M1 and the control antibody Tezepelumab can inhibit the secretion of TARC by PBMCs stimulated by TSLP.

### Example 4: Humanized engineering and functional identification of mouse anti-human TSLP monoclonal antibody

The humanization of the heavy chain of the anti-human TSLP mouse monoclonal antibody S17C7+L3E8-M1 employed a classical framework transplantation strategy (J immunol. 169, 1119-1125, 2002). The heavy chain variable region of S17C7+L3E8-M1 was compared with the human antibody germline genetic sequence in the IMGT database. The appropriate germline genetic sequence was selected to provide the framework regions 1 to 3 of the antibody (FR1+FR2+FR3), and the appropriate J region genetic sequence was selected to provide the framework region 4 (FR4). This template can be selected according to a variety of factors, such as the relative total length of the antibody, the size of the CDRs, the amino acid residues located at the junction between the framework region (FR) and the hypervariable region (CDR) of the antibody, and the homology of the whole entire sequences. The selected template can be a mixture of multiple sequences or can be a consensus template, with the aim of maintaining the appropriate conformation of the parental complementarity determining regions (CDR) as much as possible. The humanized heavy chain mutant H3B10-M1 (SEQ ID NO:24) was finally obtained. An antibody variable region gene was designed and synthesized based on the amino acid sequence of the humanized antibody, cloned into a eukaryotic expression vector and expressed with light chain L3E8-M1 as a whole antibody H3B10-M1+L3E8-M1.

Referring to Example 3.1, affinity analysis was performed on the humanized monoclonal antibody H3B10-M1+L3E8-M1 of S17C7+L3E8-M1 using Biacore X100, and the results were shown in Table 4.

**Table 4. Affinity constant for the binding of the humanized monoclonal antibody H3B10-M1+L3E8-M1 to hTSLP**

| Monoclonal antibody | Kₐ | K_{d} | K_{D} |
|---|---|---|---|
| S17C7+L3E8-M1 | 1.524E+7 | 1.767E-3 | 1.159E-10 |
| H3B10-M1+L3E8-M1 | 1.782E+7 | 1.152E-3 | 6.464E-11 |

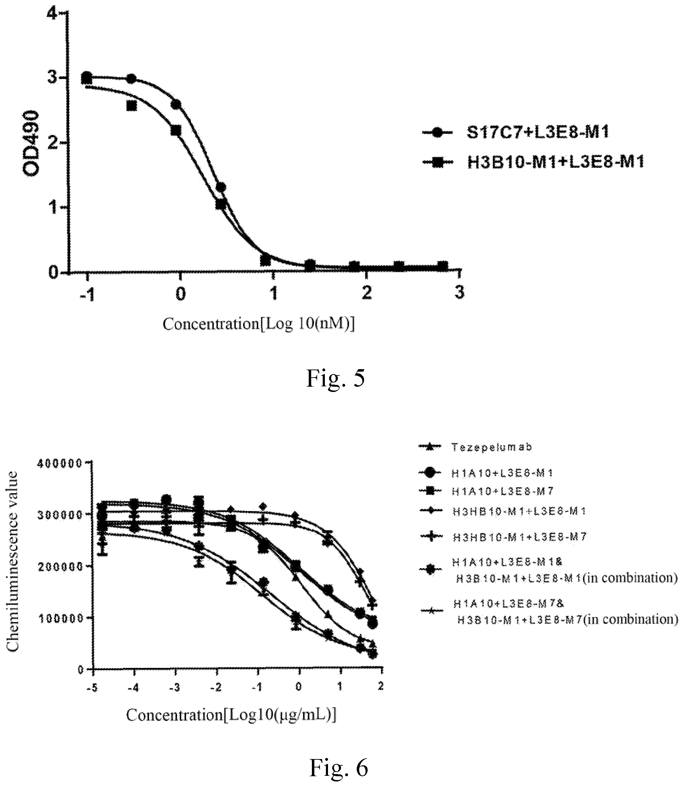

Referring to Example 3.2, the ability of the humanized monoclonal antibody H3B10-M1+L3E8-M1 to inhibit the binding of TSLP to TSLPR was analyzed by an ELISA assay. The results from the ELISA assay (FIG. 5) showed that the humanized monoclonal antibody H3B10-M1+L3E8-M1 can completely inhibit the binding of TSLP to TSLPR. The IC₅₀ values were shown in Table 5.

**Table 5. IC₅₀ values of the humanized antibody H3B10-M1+L3E8-M1 for inhibiting the binding of T TSLP to TSLPR**

| Monoclonal antibody | S17C7+L3E8-M1 | H3B10-M1+L3E8-M1 |
|---|---|---|
| IC₅₀ (nM) | 2.288 | 1.787 |

### Example 5: Optimization and identification of the common light chain L3E8-M1 5.1 Engineering of the common light chain L3E8-M1

In the process of humanization of mouse antibodies, changes in the amino acids of the framework region may lead to conformational changes in the antibody, which affects normal folding and secretion of the antibodies, damages the stability of the antibodies, and thereby affecting the expression of the antibodies. The common light chain L3E8-M1 cooperated with the heavy chain H1A10 and H3B10-M1 to express monoclonal antibodies, respectively. The expression levels were significantly reduced compared with the expression level of the patent sequence L3E8. Compared with the expression levels of other mutants of L3E8 (see L3E8-M1, with the amino acid sequence as set forth in SEQ ID NO:21; L3E8-M2, with the amino acid sequence as set forth in SEQ ID NO:25; L3E8-M3, with the amino acid sequence as set forth in SEQ ID NO:26; and L3E8-M4, with the amino acid sequence as set forth in SEQ ID NO:27 disclosed in the Chinese Patent Application No. 202010080450.1), a new mutant L3E8-M7 (SEQ ID NO:28) was constructed by introducing L78V and S80A mutations in the variable region of L3E8-M1. The antibody variable region gene was designed and synthesized based on the amino acid sequence of L3E8-M7, cloned into a eukaryotic expression vector, and was expressed respectively with H1A10 and H3B10-M1 heavy chains as whole antibodies. Referring to Example 1, the expression levels of the whole antibodies with different light chains L3E8, L3E8-M1, and L3E8-M7 were compared. The expression level of L3E8-M7 was recovered to that of parent L3E8, which was significantly superior to the expression level of L3E8-M1.

### 5.2 Affinity analysis of the humanized antibody H3B10-M1+L3E8-M7

Referring to Example 3.1, affinity analysis of H3B10-M1+L3E8-M7 was performed using Biacore X100 and the results were shown in Table 6.

**Table 6. Affinity constant for the binding of H3B 10-M1+L3E8-M7 to hTSLP**

| Monoclonal antibody | Kₐ | K_{d} | K_{D} |
|---|---|---|---|
| H3B10-M1+L3E8-M7 | 1.742E+7 | 1.056E-3 | 6.061E-11 |
| H3B10-M1+L3E8-M1 | 1.782E+7 | 1.152E-3 | 6.464E-11 |
| H1A10+L3E8-M7 | 2.053E+6 | 2.131E-5 | 1.038E-11 |

### 5.3 Inhibition of proliferation-promoting effect of TSLP on BAF3-TSLPR/IL-7Rα cells by the humanized antibody H3B10-M1+L3E8-M7

BAF3-TSLPR/IL-7Rα cells were treated referring to Example 3.4. On the day of the experiment, the cells were centrifugated and resuspended to the density of 1×10⁶ cells/mL, and added to 96-well flat bottom cell culture plates at 50µL/well. The dilutions of the recombinant anti-human TSLP monoclonal antibodies (H1A10+L3E8-M1, H1A10+L3E8-M7, H3B10-M1+L3E8-M1 and H3B10-M1+L3E8-M7) and the positive control antibody (i.e., Tezepelumab) were all started at a final concentration of 60µg/mL, with a second concentration of 30µg/mL, followed by a 6-fold gradient of each of 8 concentration gradients. The doses of the combined experimental groups (H1A10+L3E8-M1&H3B10-M1+L3E8-M1 and H1A10+L3E8-M7& H3B10-M1+L3E8-M7) were half doses of each of the monoclonal antibodies. The diluted anti-human TSLP monoclonal antibody was premixed with an equal volume of hTSLP-m-his at a final concentration of 0.4 ng/mL for 30 minutes, and then the mixture was mixed with BAF3-TSLPR/IL-7Rα cells and incubated in a total volume of 150µL/wells. After 48 hours, the cell proliferation was determined by a multifunctional microplate reader (SpectraMax^{®}I3X) using the Cell Titer-Glo^{®} Luminescent Cell Viability Assay Kit (Promega, Cat#G7570). The results of chemiluminescence readings were fitted and analyzed. The results (FIG.6) showed that both of the monoclonal antibodies H1A10+L3E8-M7 and H3B10-M1+L3E8-M7 with L3E8-M7 light chain effectively inhibited the proliferation-promoting effect of TSLP on BAF3-TSLPR/IL-7Rα cells, and that their activities were comparable to those of the monoclonal antibodies H1A10+L3E8-M1 and H3B10-M1+L3E8-M1 with L3E8-M1 light chain. Compared with the monoclonal antibody and the positive control antibody (i.e., Tezepelumab), the combined experimental groups had more significant inhibition of proliferation-promoting effect of TSLP on BAF3-TSLPR/IL-7Rα cells.

### 5.4 Effect of the humanized antibody H3B10-M1+L3E8-M7 on inhibiting the secretion of TARC by PBMCs stimulated by TSLP

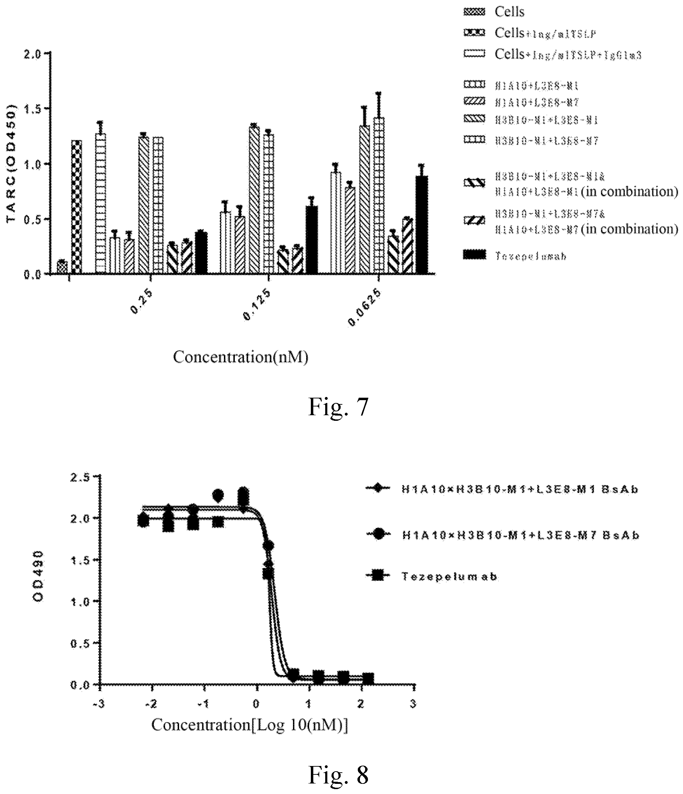

PBMC cells were obtained referring to Example 3.5. On the day of the experiment, PBMCs were resuspended in R1640 medium containing 10% (inactivated) serum to a density of 2×10⁷ cells/mL and were added to 96-well flat bottom cell culture plates (50µL/well). The dilutions of the anti-human TSLP monoclonal antibodies (H1A10+L3E8-M1, H1A10+L3E8-M7, H3B10-M1+L3E8-M1 and H3B10-M1+L3E8-M7) and the positive control antibody (i.e., Tezepelumab) were all started at a final concentration of 0.25nM, with a 2-fold gradient for a total of 3 working concentrations. The doses of the combined experimental groups (H1A10+L3E8-M1&H3B10-M1+L3E8-M1 and H1A10+L3E8-M7& H3B10-M1+L3E8-M7) were half doses of each of the monoclonal antibodies. No TSLP control (cells), no antibody control (cells +1ng/mL TSLP), and IgG1m3 isotype antibody control (cells +1ng/mL TSLP+IgG1m3) were set. The diluted recombinant anti-human TSLP monoclonal antibody was premixed with an equal volume of hTSLP-m-his at a final concentration of 1 ng/mL for 30 minutes, and then the mixture was added to the cells for mixing, with a total volume of 150µL/well. The mixture was incubated at 37°C, 5% CO₂ for 24 hours. The supernatant was then taken and the TARC was detected using the humanized antibody TARC matched ELISA antibody pair set (Sino Biological Inc., Cat# SEK10233) based on an ELISA assay. The chromogenic solution was a TMB high-sensitivity substrate solution (Biolegend, Cat# 421501). The optical density value of OD450 was determined using a microplate reader (Biotek, Cat# ELX800). The readings were analyzed. The results (FIG. 7) showed that both of the monoclonal antibodies H1A10+L3E8-M7 and H3B10-M1+L3E8-M7 with L3E8-M7 light chain effectively inhibited the secretion of TARC by PBMCs stimulated by TSLP, and that their activities were comparable to those of the monoclonal antibodies H1A10+L3E8-M1 and H3B10-M1+L3E8-M1 with L3E8-M1 light chain. Compared with the monoclonal antibodies and the positive control antibody (i.e., Tezepelumab), the combined experimental groups had more significant inhibition of the secretion of TARC by PBMCs stimulated by TSLP.

### Example 6: Preparation and identification of anti-human TSLP bispecific antibodies

### 6.1 Preparation of anti-human TSLP bispecific antibodies

The nucleotide sequences encoding the heavy chain variable regions H1A10 and H3B10-M1 of the monoclonal antibodies against two different epitopes of human TSLP were respectively cloned into suitable eukaryotic expression vectors to construct heterodimers based on the common light chain L3E8-M7. That is, the nucleotide sequence encoding the H1A10 heavy chain variable region was cloned into a eukaryotic expression vector fused with the nucleotide sequence encoding the IgG1 constant region IgG1m3-K with Knob mutation; the nucleotide sequence encoding the H3B10-M1 heavy chain variable region was cloned into a eukaryotic expression vector containing the nucleotide sequence encoding the IgG1 constant region IgG1m3-H with Hole mutation; and the nucleotide sequence encoding the common light chain L3E8-M7 variable region was cloned into a eukaryotic expression vector fused with the nucleotide sequence encoding the human light chain constant region CK.

The three constructed eukaryotic expression vectors expressing H1A10-IgG1m3-K, H3B10-M1-IgG1m3-H and L3E8-M7-CK were co-transfected into HEK293F cells using liposomes, and the cells were cultured in suspension in a serum-free medium for 3-5 days. The culture supernatant was harvested by centrifugation. The bispecific antibodies in the culture supernatant were purified using a Protein A/G affinity chromatography column (e.g., Mabselect SURE, GE Inc., etc). The recombinant protein preservation buffer was then replaced with a BS buffer (pH 7.0) or other suitable buffers using a desalting column (e.g., Hitrap desaulting, GE Inc., etc). The desalted protein solution was purified by a size exclusion chromatography (SEC) using Superdex 200 (GE) to obtain the protein of interest. If necessary, the antibody samples can be sterilized by filtration and then stored in aliquots at -20°C for use.

### 6.2 Affinity analysis of anti-human TSLP bispecific antibodies

Referring to Example 3.1, the affinities of the anti-human TSLP bispecific antibody (BsAb) molecules and the monoclonal antibody molecules binding to human TSLP (hTSLP) and cynomolgus monkey TSLP (mfTSLP) was analyzed using Biacore X 100. The results were shown in Tables 7 and 8.

**Table 7. Affinity constants for the binding of anti-human TSLP antibodies to hTSLP**

| Antibody | Kₐ | K_{d} | K_{D} |
|---|---|---|---|
| H3B10-M1+L3E8-M7 | 1.477E+7 | 1.176E-3 | 7.963E-11 |
| H1A10+L3E8-M7 | 2.828E+6 | 3.999E-5 | 1.414E-11 |
| H1A10×H3B10-M1+L3E8-M7 BsAb | 1.741E+7 | 1.118E-4 | 6.421E-12 |
| H1A10×H3B10-M1+L3E8-M1 BsAb | 1.618E+7 | 1.313E-4 | 8.115E-12 |

**Table 8. Affinity constants for the binding of anti-human TSLP antibodies to mfTSLP**

| Antibody | Kₐ | K_{d} | K_{D} |
|---|---|---|---|
| H1A10+L3E8-M7 | 4.241E+6 | 1.205E-2 | 2.842E-9 |
| H1A10×H3B10-M1+L3E8-M7 BsAb | 7.132E+6 | 1.963E-2 | 2.753E-9 |
| H1A10×H3B10-M1+L3E8-M1 BsAb | 1.004E+7 | 2.497E-2 | 2.487E-9 |

### 6.3 Inhibition of the binding of TSLP to TSLPR by anti-human TSLP bispecific antibodies

Referring to Example 3.2, the abilities of anti-TSLP bispecific antibodies to inhibit the binding of TSLP to TSLPR were analyzed by an ELISA assay. The results from the ELISA assay (FIG. 8) showed that the abilities of the anti-human TSLP bispecific antibodies H1A10×H3B10-M1+L3E8-M1 BsAb and H1A10×H3B10-M1+L3E8-M7 BsAb to inhibit the binding of TSLP to TSLPR were comparable to that of Tezepelumab. The IC₅₀ values were shown in Table 9.

**Table 9. IC₅₀ values of anti-human TSLP bispecific antibodies for inhibiting the binding of TSLP to TSLPR**

| Antibody | H1A10×H3B10-M1 +L3E8-M1 BsAb | H1A10×H3B10-M1 +L3E8-M7 BsAb | Tezepelumab |
|---|---|---|---|
| IC₅₀(nM) | 1.906 | 2.171 | 1.724 |

### 6.4 Inhibition of the proliferation-promoting effect of TSLP on BAF3-TSLPR/IL-7Rα cells by anti-human TSLP bispecific antibodies

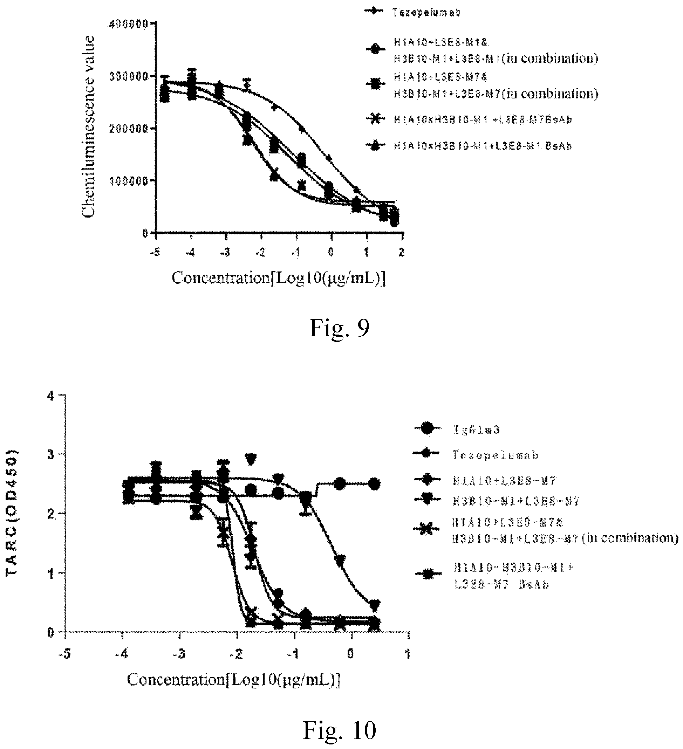

Referring to Example 5.3, the cell proliferation was determined using the Cell Titer-Glo^{®} Luminescent Cell Viability Assay Kit (Promega, Cat#G7570) and the values were read by a multifunctional microplate reader (SpectraMax^{®}I3X). The results (FIG. 9) showed that compared with the combined experimental groups (H1A10+L3E8-M1&H3B10-M1+L3E8-M1 and H1A10+L3E8-M7&H3B10-M1+L3E8-M7) and the positive control antibody (i.e., Tezepelumab), the anti-human TSLP bispecific antibodies (H1A10×H3B10-M1+L3E8-M1 BsAb and H1A10×H3B10-M1+L3E8-M7 BsAb) had more significant inhibition of the proliferation-promoting effect of TSLP on BAF3-TSLPR/IL-7Rα cells.

### 6.5 Effect of anti-human TSLP bispecific antibody on inhibiting the secretion of TARC by PBMCs stimulated by TSLP

PBMC cells were obtained referring to Example 3.5. On the day of the experiment, PBMCs were resuspended in R1640 medium containing 10% (inactivated) serum to a density of 2×10⁷ cells/mL and were added to 96-well flat bottom cell culture plates (50µL/well). The dilutions of the anti-human TSLP monoclonal antibodies H1A10+L3E8-M7 and H3B10-M1+L3E8-M7, the anti-human TSLP bispecific antibody H1A10×H3B10-M1+L3E8-M7 BsAb and the positive control antibody (i.e., Tezepelumab) were all started at a final concentration of 0.25µg/mL, with a 4-fold dilution for the first 3 points, a 3-fold dilution for the middle 4^{th} -6^{th} points, and a 5-fold dilution for 7^{th} -9^{th} points. The dose of the combined experimental group (H1A10+L3E8-M7& H3B10-M1+L3E8-M7) was half dose of each of the monoclonal antibodies. IgG1m3 was used as the isotype antibody control. The diluted anti-human TSLP antibody was premixed with an equal volume hTSLP-m-his at a final concentration of 1 ng/mL for 30 minutes, and then the mixture was added to the cells for mixing, with a total volume of 150µL/well. The mixture was incubated at 37°C, 5% CO₂ for 24 hours. The supernatant was then taken and the TARC was detected using the humanized antibody TARC matched ELISA antibody pair set (Sino Biological Inc., Cat# SEK10233) based on an ELISA assay. The chromogenic solution was a TMB high-sensitivity substrate solution (Biolegend, Cat# 421501). The optical density value of OD450 was determined using a microplate reader (Biotek, Cat# ELX800). The readings were fitted and analyzed. The results showed (FIG. 10) that compared with the monoclonal antibody experimental groups and the positive control antibody (i.e., Tezepelumab), the bispecific antibody and the combined experimental group had more significant inhibition of the secretion of TARC by PBMCs stimulated by TSLP.

### Example 7: Dual epitope analysis of anti-human TSLP bispecific antibody

### 7.1 Dual epitope identification of anti-human TSLP bispecific antibody

The binding epitopes of anti-TSLP antibody were analyzed by surface plasmon resonance technique using Biacore T200.

### Reagent Consumables

An amino coupling kit (BR-1000-50), a His kit (28995056), a S-series CM5 chip (BR100530), and 10×HBS-EP ( pH 7.4, BR100669) and so on were all available from GE healthcare.

### Chip coupling

The surface of the carboxylated CM5 chip was activated with 1-ethyl-3-(3-dimethylaminopropyl) carbodiimide hydrochloride (EDC) and N-hydroxysuccinimide (NHS) according to the instructions in the amino coupling kit. An anti-His antibody (capture antibody) was diluted to 25µg/mL with 10 mM sodium acetate (pH 5.0), and then injected at a flow rate of 10µL/min to achieve a coupling amount of approximately 12,000 response units (RU). After the injection of capture antibody, 1 M ethanolamine was injected to block the unreacted groups.

### Epitope analysis

The recombinant protein hTSLP1-m-His was diluted to 2µg/mL with 1×HBS-EP and injected at 10µL/min for 12 seconds to ensure that about 25RU of the hTSLP1-m-His was captured by the anti-His antibody. The anti-human TSLP monoclonal antibodies H3B10-M1+L3E8-M7, H1A10+L3E8-M7 and Tezepelumab, and the anti-human TSLP bispecific antibody H1A10×H3B10-M1+L3E8-M7 (H1A10×H3B10-M1+L3E8-M7 BsAb) were diluted to 45µg/ml with 1×HBS-EP, respectively. Different antibodies were sequentially injected using a double injection mode of Biacore T200. That is, the first antibody flowed through the surface of the chip capturing the antigen hTSLP-m-His at a flow rate of 30µL/min, followed by the injection of the second antibody. Each of the antibodies was injected for 120 seconds. The surface of the chip was regenerated at the end of the injection by injecting 1.7mM glycine-HCl at 10µL/min for 30s. The capture level reporting point for hTSLP1-m-His was zeroed using Biacore T200 evaluation software version 3.0, and then the RU values were plotted against times using GraphPad software (connecting lines).

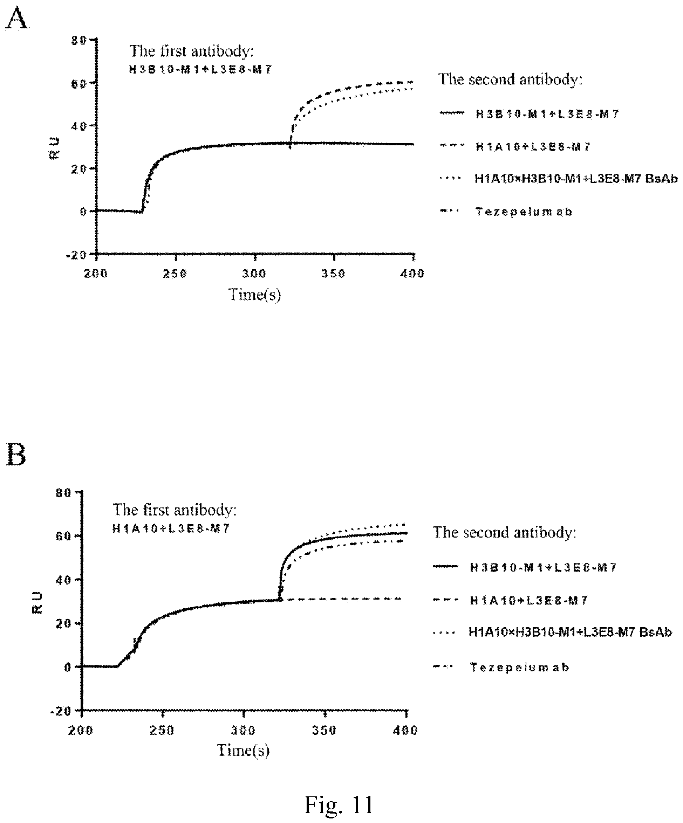

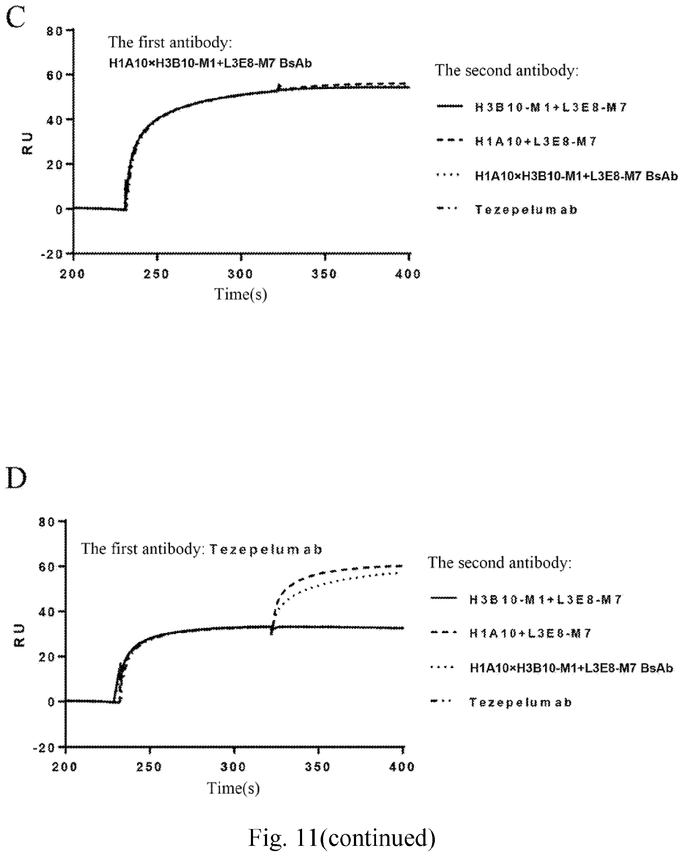

The results of the epitope analysis were shown as follows: 1) No binding signal of Tezepelumab was detected when first injecting H3B10-M1+L3E8-M7 and then Tezepelumab (FIG. 11A, Table 10); similarly, no binding signal of H3B10-M1+L3E8-M7 was detected when first injecting Tezepelumab and then H3B10-M1+L3E8-M7 (FIG. 11D, Table 10), indicating that the epitope of TSLP bound by H3B10-M1+L3E8-M7 was overlapped with that of TSLP bound by Tezepelumab. 2) The binding signals were significantly overlapped when first injecting H3B10-M1+L3E8-M7 and then H1A10+L3E8-M7 (FIG. 11A, Table 10); the binding signals were significantly overlapped when first injecting H1A10+L3E8-M7 and then H3B10-M1+L3E8-M7 (FIG. 11B, Table 10), indicating that H3B10-M1+L3E8-M7 and H1A10+L3E8-M7 recognized different TSLP epitopes. 3) After the bispecific antibody H1A10×H3B10-M1+L3E8-M7 (H1A10×H3B10-M1+L3E8-M7 BsAb) binding to TSLP, neither H3B10-M1+L3E8-M7 nor H1A10+L3E8-M7 can continue to bind to TSLP (FIG. 11C, Table 10), indicating that THE bispecific antibody H1A10×H3B10-M1 recognized the dual epitopes of TSLP.

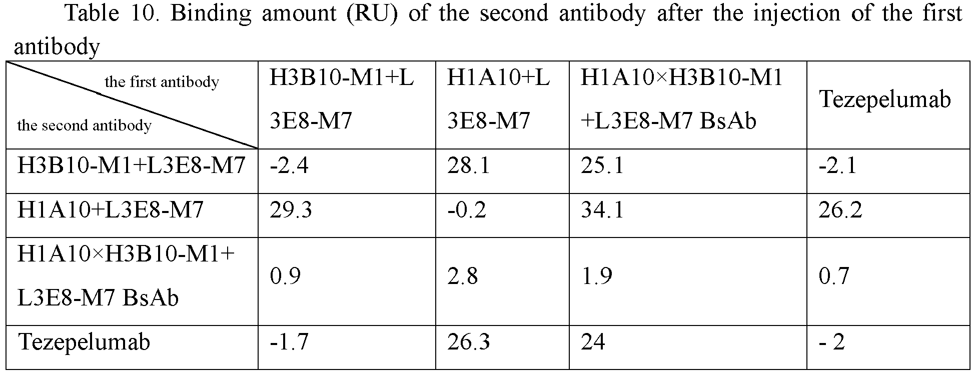

### 7.2 Binding epitope analysis of anti-human TSLP antibody

TSLP binding epitope of anti-human TSLP monoclonal antibody H1A10+L3E8-M1 was analyzed. In order to study the binding epitope of humanized monoclonal antibody H1A10+L3E8-M1 to human TSLP, the recombinant H1A10+L3E8-M1 Fab and the recombinant human TSLP were prepared from CHO cells to form a H1A10+L3E8-M1 Fab-TSLP complex. The complex crystal was formed under the conditions of 0.2M sodium formate and 20% PEG3350. X-ray diffraction experiment was performed on the complex crystal, and data with a resolution 2.23 Å were collected. The crystal structure of the TSLP antigen-antibody complex was analyzed by the molecular replacement method. The densities of the light and heavy chains and the electron density of the antigen in the H1A10+L3E8-M1 Fab-TSLP complex structure model were clear, and the values of R-work and R-free were 0.198 and 0.260, respectively.

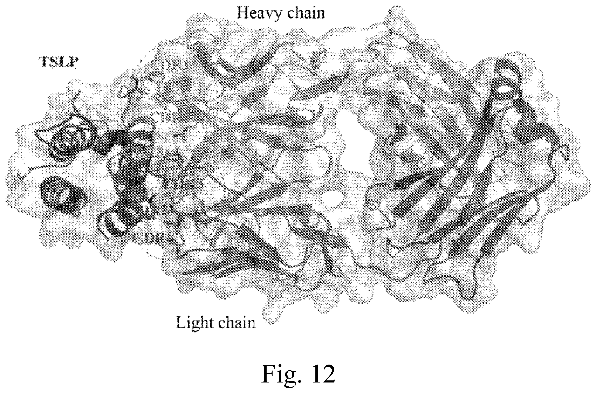

The interaction area of TSLP with H1A10+L3E8-M1 Fab was about 895.1 Å2, in which the interaction area of TSLP with the heavy chain was about 612.5 Å2 and the interaction area of TSLP with the light chain was about 282.6 Å2. The interactions mainly involve two helices of the amino acids at positions 41-53 and the amino acids at positions 95-114 of TSLP, whereas HCDR1, HCDR2, and HCDR3 of the heavy chain variable region of the antibody, as well as LCDR1and LCDR3 of the light chain variable region of the antibody participated in the recognition of TSLP (FIG. 12). The key amino acid sites of the heavy chain involved in TSLP binding were N31, Q50, D57, Y101, G104, D55, and D57. The key amino acid sites of the light chain involved in the TSLP binding were N33, Y92, and Y95. The mode of the interaction of H1A10+L3E8-M1 Fab with TSLP included hydrogen bonds, salt bonds, van der Waals forces, and hydrophobic interactions (see Table 11).

**Table 11. Amino acid residues involved in H1A10+L3E8-M1 Fab-hTSLP binding**

| TSLP | | Heavy chain | | Light chain | | Interaction |
|---|---|---|---|---|---|---|
| Region | Residue | Region | Residue | Region | Residue | |
| α-A | A42 | CDR3 | Y101 | | | Hydrophobic interaction |
| | S45 | | | CDR1 | S32 | Hydrogen bond |
| | T46 | CDR3 | G104 | | | Hydrogen bond |
| | K49 | | | CDR1 | S30 | Van der Waals interaction |
| | | | | CDR1 | S32 | Van der Waals interaction |
| | D50 | | | CDR1 | N33 | Hydrogen bond |
| | T53 | | | CDR1 | S30 | Van der Waals interaction |
| BC ring | K95 | CDR2 | D59 | | | Van der Waals interaction |
| | M97 | | | CDR3 | S93 | Van der Waals interaction |
| | | | | CDR3 | S94 | Van der Waals interaction |
| | | | | CDR3 | Y95 | Hydrogen bond |
| α-C | M100 | CDR2 | Q50 | | | Hydrogen bond |
| | | CDR1 | W33 | | | Hydrogen bond |
| | | CDR2 | D59 | | | Van der Waals interaction |
| | | | | CDR3 | Y95 | Hydrogen bond |
| | | | | CDR3 | Y95 | Van der Waals interaction |
| | | | | CDR3 | W92 | Van der Waals interaction |
| | K101 | CDR2 | D55 | | | Salt bridge |
| | | CDR2 | D57 | | | Salt bridge |
| | | CDR3 | Y101 | | | Hydrogen bond |
| | | CDR3 | Y106 | | | Hydrogen bond |
| | | | | CDR1 | N33 | Hydrogen bond |
| | | | | CDR3 | W92 | Hydrogen bond |
| | | | | CDR3 | W92 | Van der Waals interaction |
| | K103 | CDR2 | P52 | | | Van der Waals interaction |
| | A104 | CDR3 | Y106 | | | Hydrogen bond |
| | A105 | CDR3 | Y101 | | | Hydrophobic interaction |
| | I108 | CDR3 | Y101 | | | Hydrophobic interaction |
| | | CDR1 | Y32 | | | Hydrogen bond |
| | W109 | CDR3 | Y101 | | | Hydrophobic interaction |
| CD ring | S114 | CDR1 | N31 | | | Hydrogen bond |
| | | CDR2 | P52 | | | Van der Waals interaction |

The interaction of IL-7R with TSLP mainly involves the two helices of the amino acids at positions 41-49 and the amino acids at positions 97-109 of TSLP ^{[28]}. The TSLP epitopes bound by H1A10+L3E8-M1 mainly involve the amino acids at positions 41-53 and the amino acids at positions 95-114 of TSLP. The TSLP epitope bound by H1A10+L3E8-M1 was overlapped with the epitope TSLP bound by IL-7R. H1A10-L3E8-M1 inhibited the binding of TSLP to IL-7R. L3E8-M1 underwent a two-point mutation to become L3E8-M7, with the mutation sites L79V and S81A. These two sites were part of the structural stacking and spatially distant from the CDRs. The amino acids at these mutation positions were not involved in the binding to TSLP and did not affect the key amino acids binding to TSLP. Theoretically, it can be inferred that L3E8-M7 acts with TSLP in the same way that L3E8-M1 acts with TSLP, *i.e.,* H1A10+L3E8-M7 and H1A10+L3E8-M1 act with TSLP in the same way.

The interaction of Tezepelumab with TSLP mainly involves the amino acids at positions 65-78 and the amino acids at positions 150-153 of TSLP. The interaction of TSLPR with TSLP mainly involves the amino acids at positions 60-69, the amino acids at positions 142-152 and the amino acids at positions 153-158 of TSLP ^{[28]}. That is, the TSLP epitope bound by Tezepelumab was overlapped with the TSLP epitope bound by TSLPR. The epitope analysis data of various monoclonal and bispecific antibodies in Section 7.1 showed that the TSLP epitope bound by H3B10-M1+L3E8-M7 was overlapped with the TSLP epitope bound by Tezepelumab. It can be inferred that the TSLP epitope bound by H3B10-M1+L3E8-M7 was overlapped with the TSLP epitope bound by TSLPR.

### Example 8: Blocking of human TSLP: TSLPR:IL-7Rα complex formation by anti-human TSLP antibodies

TSLP first binds to TSLPR on cell surface to form a binary complex, and then recruits IL-7Rα to form TSLP: TSLPR:IL-7Rα ternary complex before transducing the downstream signaling pathways. TSLP can bind to TSLPR with high affinity (K_{D}=32nM). The interaction of TSLP with IL-7Rα was weak. However, IL-7Rα can bind to TSLP: TSLPR complex with high affinity (K_{D}=29nM) ^{[28]}. Functional anti-TSLP antibodies function primarily by blocking the formation of TSLP: TSLPR: IL-7Rα ternary complex in two ways: i) blocking the binding of TSLPR to TSLP, and ii) blocking the binding of IL-7Rα to TSLP: TSLPR binary complex.

### Reagent consumables

The reagent consumables, such as an amino coupling kit (BR-1000-50), a mouse antibody capture kit (BR100838), a S-series CM5 chip (BR100530), and 10×HBS-EP at pH 7.4 (BR100669) were all available from GE healthcare.

### Chip coupling

The surface of the carboxylated CM5 chip was activated with 1-ethyl-3-(3-dimethylaminopropyl) carbodiimide hydrochloride (EDC) and N-hydroxysuccinimide (NHS) according to the instructions in the amino coupling kit. An anti-mouse Fc (mFc) antibody (capture antibody) was diluted to 25µg/mL with 10 Mm sodium acetate (pH 5.0), and then injected at a flow rate of 10µL/min to achieve a coupling amount of approximately 10,000 response units (RU). After the injection of capture antibody, 1 M ethanolamine was injected to block the unreacted groups.

### 8.1 Blocking the binding of human TSLPR to human TSLP by anti-human TSLP antibodies

The extracellular domain of human TSLP receptor (hTSLPR-ECD, SEQ ID NO:42) was expressed referring to Example 1. The recombinant protein hTSLP1-m-mFc was diluted to 2µg/mL with 1×HBS-EP buffer, and was injected at 10µL/min for 12s. Approximately 80RU of hTSLP1-m-mFc was captured by the anti-mFc antibody. Anti-hTSLP antibodies (H3B10-M1+L3E8-M7, H1A10+L3E8-M7, and H1A10×H3B10-M1+L3E8-M7 BsAb) were diluted to 50µg/mL with 1×HBS-EP buffer, and hTSLPR was diluted to 100µg/mL. Two molecules were sequentially injected using a double injection mode of Biacore T200. That is, the anti-TSLP antibody (the first analyte) flowed through the surface of the chip capturing the antigen hTSLP1-m-mFc recombinant protein at a flow rate of 30µL/min, followed by the injection of the same antibody or hTSLPR-ECD (the second analyte). Each of the molecules was injected for 90s. The surface of the chip was regenerated at the end of the injection by injecting 10mM glycine-HCl (pH 1.7) at 10µL/min for 30s. The Y-axis reporting points for the double baselines were zeroed using Biacore T200 evaluation software version 3.0, and then the RU values were plotted against times using GraphPad software.

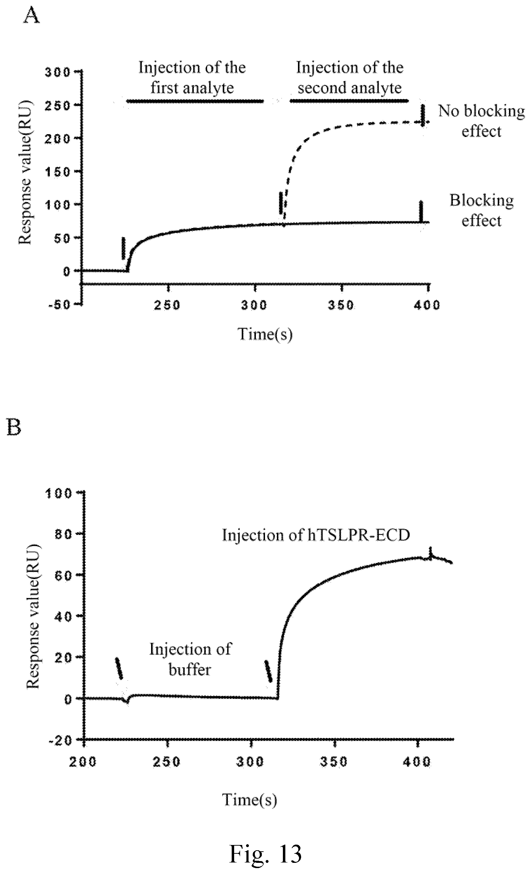

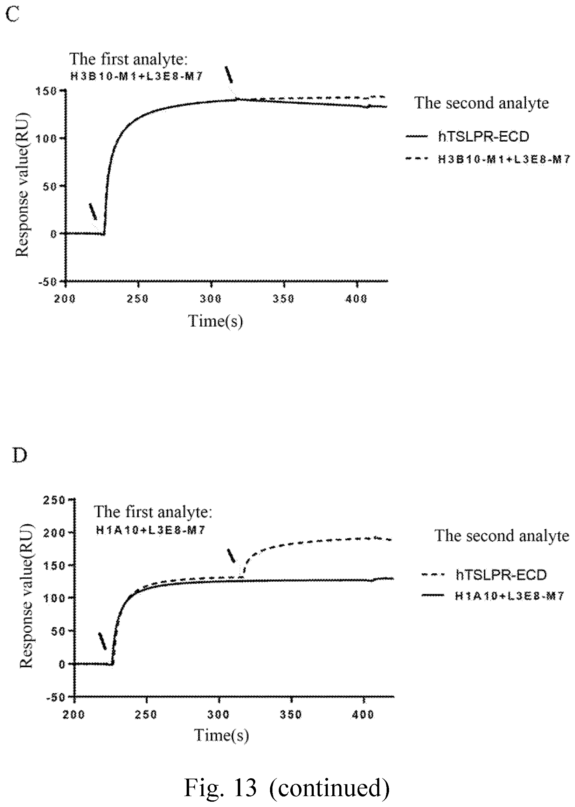

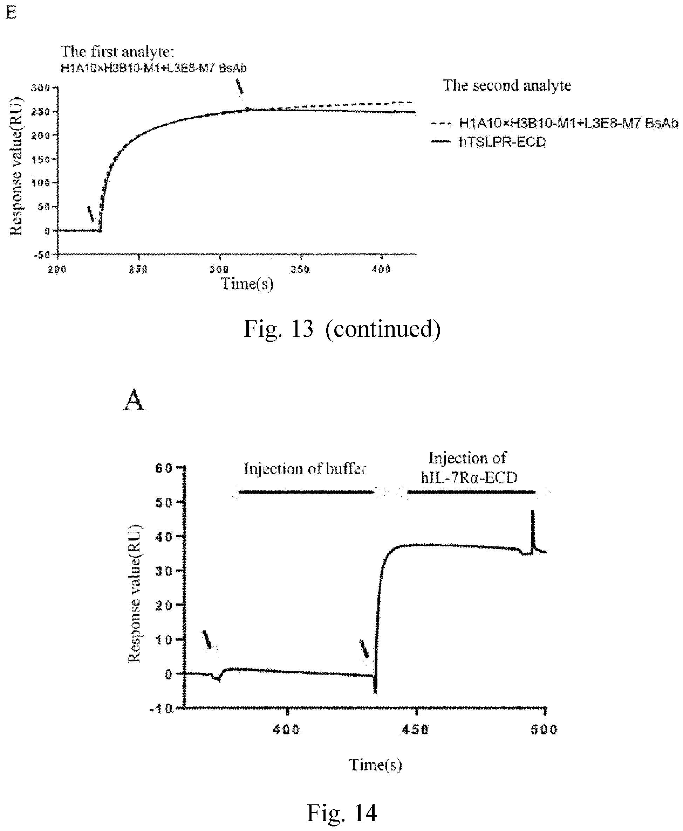

FIG. 13A shows the basic principle of protein blocking analysis using Biacore T200. The results were shown as follows: 1) When hTSLPR-ECD was injected alone, the binding amount of hTSLPR-ECD was 68.1RU (FIG. 13B and Table 12). 2) No binding signal of hTSLPR-ECD was detected when first injecting H3B10-M1+L3E8-M7 and then hTSLPR-ECD (FIG. 13C and Table 12), indicating that H3B10-M1+L3E8-M7 blocked the binding of hTSLPR-ECD to the recombinant protein hTSLP1-m-mFc. 3) When first injecting H1A10+L3E8-M7 and then hTSLPR-ECD, the binding signal of hTSLPR-ECD was similar to that of hTSLPR-ECD alone (FIG. 13D and Table 12), indicating that H1A10+L3E8-M7 did not block the binding of hTSLPR-ECD to the recombinant protein hTSLP1-m-mFc. 4) hTSLPR-ECD can no longer continue to bind to the recombinant protein hTSLP1-m-mFc after the binding of H1A10×H3B10-M1+L3E8-M7 BsAb to the recombinant protein hTSLP1-m-mFc (FIG. 13E and Table 12), indicating that H1A10×H3B10-M1+L3E8-M7 BsAb can block the binding of hTSLPR-ECD to the recombinant protein hTSLP1-m-mFc.

**Table 12. Binding amount (RU) of hTSLPR-ECD after the injection of anti-hTSLP antibodies**

| | Binding amount of HTSLPR-ECD |
|---|---|
| Buffer | 68.1 |
| H3B10-M1+L3E8-M7 | -6 |
| H1A10+L3E8-M7 | 59.1 |
| H1A10×H3B10-M1+L3E8-M7 BsAb | -1.7 |

### 8.2 Blocking the binding of human IL-7Rα to human TSLP: TSLPR complex by anti-human TSLP antibodies

The extracellular domain of human IL-7Rα (hIL-7Rα-ECD , SEQ ID NO:43) was expressed referring to Example 1. The recombinant proteins hTSLP1-m-mFc and hTSLPR-ECD were respectively diluted to 2µg/mL and 100µg/mL with 1×HBS-EP buffer. hTSLP1-m-mFc and hTSLPR-ECD were sequentially injected for 12s and 60s so as to form hTSLP: hTSLPR complex on the surface of the CMS chip. Anti-hTSLP antibodies (H3B10-M1+L3E8-M7, H1A10+L3E8-M7 and H1A10×H3B10-M1+L3E8-M7 BsAb) were diluted to 50µg/mL with 1×HBS-EP buffer, and hIL-7Rα-ECD was diluted to 200µg/mL. Two molecules were sequentially injected using a double injection mode of Biacore T200. That is, the anti-TSLP antibody flowed through the surface of the chip capturing the hTSLP: hTSLPR complex at a flow rate of 30µL/min, followed by the injection of hIL-7Rα-ECD. Each of the molecules was injected for 60s. The surface of the chip was regenerated at the end of the injection by injecting 10mM glycine-HCl (pH 1.7) at 10µL/min for 30s. The Y-axis reporting points for the double baselines were zeroed using Biacore T200 evaluation software version 3.0, and then the RU values were plotted against times using GraphPad software.

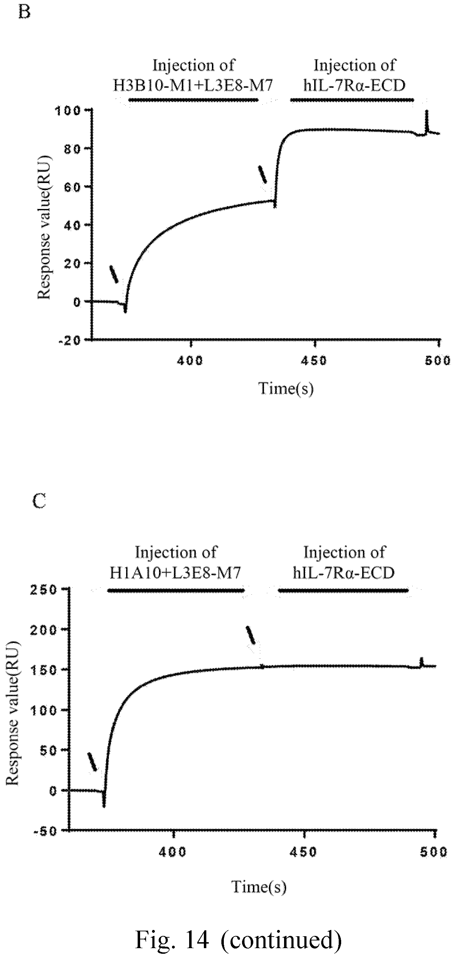

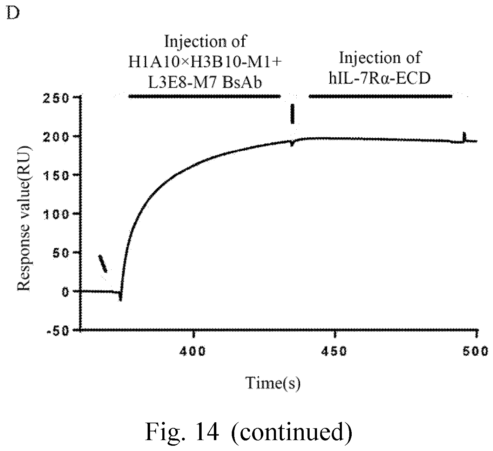

The results were shown as follows: 1) When hIL-7Rα-ECD was injected alone, the response value of the binding of hIL-7Rα-ECD to the hTSLP: hTSLPR complex was 36.2RU (FIG. 14A and Table 13). 2) By first injecting H3B10-M1+L3E8-M7, and then hIL-7Rα-ECD, the binding signal of hIL-7Rα-ECD was similar to that of hIL-7Rα-ECD alone (FIG. 14B and Table 13), indicating that H3B10-M1+L3E8-M7 did not block the binding of hIL-7Rα-ECD to the hTSLP: hTSLPR complex. 3) No binding signal of hIL-7Rα-ECD was detected when first injecting H1A10+L3E8-M7, and then hIL-7Rα-ECD (FIG. 14C and Table 13), indicating that H1A10+L3E8-M7 can block the binding of hIL-7Rα-ECD to the hTSLP: hTSLPR complex. 4), hIL-7Rα-ECD cannot bind to the hTSLP: hTSLPR complex after the binding of H1A10×H3B10-M1+L3E8-M7 BsAb to hTSLP (FIG. 14D and Table 13), indicating that H1A10×H3B10-M1+L3E8-M7 BsAb can block the binding of hIL-7Rα-ECD to the hTSLP: hTSLPR complex.

**Table 13. Binding amount (RU) of hIL-7Rα-ECD after the injection of anti-TSLP antibodies**

| | Binding amount of hIL-7Rα-ECD |
|---|---|
| Buffer | 36.2 |
| H3B10-M1+L3E8-M7 | 36.5 |
| H1A10+L3E8-M7 | 1.7 |
| H1A10×H3B10-M1+L3E8-M7 BsAb | 3.1 |

Exemplary embodiments of the present invention have been described above. However, those skilled in the art can modify or improve the exemplary embodiments described herein without departing from the spirit and scope of the present application, and variations or equivalents derived therefrom are also within the scope of the present application.

### Reference

1. Leonard, W. J. (2002) TSLP is now revealed to be an important regulator of DC-mediated control of TH2-based human allergic responses, identifying a potentially new species-specific function for this cytokine. Nat Immunol 3, 605-607.
2. Liu, Y J., Soumelis, V, Watanabe, N., et al. (2007) TSLP: an epithelial cell cytokine that regulates T cell differentiation by conditioning dendritic cell maturation. Annual Review of Immunology 25, 93-219.
3. Ziegler, S.F., Artis, D. (2010) Sensing the outside world: TSLP regulates barrier immunity. Nat Immunol 11, 289-293.
4. Allakhverdi, Z., Comeau, M.R., Jessup, H.K., et al. (2009) Thymic stromal lymphopoietin as a mediator of crosstalk between bronchial smooth muscle and mast cells. Journal of Allergy and Clinical Immunology 123,958-960.
5. Allakhverdi, Z., Comeau, M.R., Jessup, H.K., et al. (2007) Thymic stromal lymphopoietin is released by human epithelial cells in response to microbes, trauma, or inflammation and potently activates mast cells. J Exp Med 204, 253-258.
6. Lee, H.C., Ziegler, S.F. (2007) Inducible expression of the proallergic cytokine thymic stromal lymphopoietin in airway epithelial cells is controlled by NFκB. Proceedings of the National Academy of Sciences 104, 914-919.
7. Ying, S., O"Connor, B., Ratoff, J., et al. (2005) Expression and cellular provenance of thymic stromal lymphopoietin and chemokines in patients with severe asthma and chronic obstructive pulmonary disease. Journal of Allergy and Clinical Immunology 115, S9.
8. Gounni, A.S. (2007) Constitutive and inducible thymic stromal lymphopoietin expression in human airway smooth muscle cells: role in chronic obstructive pulmonary disease. American Journal of Physiology Lung Cellular & Molecular Physiology 293,375-382.
9. Ying, S., O"Connor, B., Ratoff, J., et al. (2005). Thymic stromal lymphopoietin expression is increased in asthmatic airways and correlates with expression of th2-attracting chemokines and disease severity. The Journal of Immunology 174, 8183-8190.
10. Kashyap, M., Rochman, Y, Spolski, R., Samsel, L., et al. (2011) Thymic stromal lymphopoietin is produced by dendritic cells. The Journal of Immunology 187(3), 13-24.
11. Soumelis, V, Reche, P. A., Kanzler, H., et al. (2002) Human epithelial cells trigger dendritic cell mediator allergic inflammation by producing TSLP. Nat Immunol 3, 673-680.
12. Watanabe, N., Hanabuchi, S., Soumelis, V, et al. (2004) Human thymic stromal lymphopoietin promotes dendritic cell-mediated CD4+ T cell homeostatic expansion. Nat Immunol 5,426-434.
13. Moon, P.D., Choi, I.H., Kim, H.M. (2011) Naringenin suppresses the production of thymic stromal lymphopoietin through the blockade of RIP2 and caspase-1 signal cascade in mast cells. European Journal of Pharmacology 671,128-132.
14. Barton, G.M., Farr, A.G., et al. (2008) A mechanism for the initiation of allergen-induced T helper type 2 responses. Nature Immunology 9(3), 310-318.
15. He R, Geha, R.S. (2010) Thymic stromal lymphopoietin. Ann NYAcad Sci 1183, 13-24.
16. Oyoshi, M.K., Larson, R.P., Ziegler, S.F., et al. (2010) Mechanical injury polarizes skin dendritic cells to elicit a T (H) 2 response by inducing cutaneous thymic stromal lymphopoietin expression. Journal of Allergy & Clinical Immunology 126, 976-984; e975.
17. Hai yan Tu, Xin Chen, Jing Li. (2007) Signal transduction in respiratory syncytial virus infection-induced thymic stromal lymphopoietin expression in human epithelial cells. Journal of Southern Medical University 27(10), 1581-1583.
18. Medoff, B.D., Landry, A. L., Wittbold, K.A., et al. (2009) CARMA3 mediates lysophoshatidic acid-stimulated cytokine secretion by bronchial epithelial cells. American Journal of Respiratory Cell and Molecular Biology 2009(40), 286-294.
19. Park, L.S., Martin, U., Garka, K., et al. (2000) Cloning of the murine thymic stromal lymphopoietin (TSLP) receptor: formation of a functional heteromeric complex requires interleukin 7 receptor. The Journal of Experimental Medicine 192, 659-670.
20. Kenneth Verstraete, Frank Peelman, Harald Braun,et al.(2017) Structure and antagonism of the receptor complex mediated by human TSLP in allergy and asthma. Nature Communications 8, 14937.
21. GINA Report, Global Strategy for Asthma Management and Prevention. Available from: http ://www.ginasthma.com.
22. von Bülow, Anna, Kriegbaum, M., Backer, V, et al. (2014) The prevalence of severe asthma and low asthma control among Danish adults. The Journal of Allergy and Clinical Immunology: In Practice 2(6), 759-673.
23. Reche, P.A., Soumelis, V.M., Gorman. D.M., et al. (2001) Human thyroid stromal lymphopoietin preferentially stimulates myeloid cells. The Journal of Immunology 167, 336-343.
24. Zhou, B., Comeau, M.R., De, S.T., et al. (2005) Thymic stromal lymphopoietin as a key initiator of allergic airway inflammation in mice. Nat Immunol 6, 1047-1053.
25. Zhuang gui Chen, Tian tuo Zhang, Hong tao Li, et al. (2013) Neutralization of TSLP inhibits airway remodeling in a murine model of allergic asthma induced by chronic exposure to house dust mite. PLoS One 8, e51268.
26. He, R., Oyoshi, M.K., Garibyan, L., et al. (2008). TSLP acts on infiltrating effector T cells to drive allergic skin inflammation. Proc Natl Acad Sci USA 105(33), 11875-11880.
27. Corren, J., Parnes, J. R., Wang, L., et al. (2017) Tezepelumab in Adults with Uncontrolled Asthma. New England Journal of Medicine 377(10), 936-946.
28.Verstraete K, Peelman F, Braun H, et al. 2017. Structure and antagonism of the receptor complex mediated by human TSLP in allergy and asthma. Nat Commun [J], 8: 14937.

## Claims

1. A bispecific antibody comprising a first binding fragment and a second binding fragment that bind to non-overlapping epitopes of human thymic stromal lymphopoietin (TSLP).

2. The bispecific antibody according to claim 1, wherein the bispecific antibody is capable of blocking the binding of human TSLP to human TSLP receptor (TSLPR), and the bispecific antibody is capable of blocking the binding of human IL-7 receptor alpha chain (IL-7Rα) to human TSLP: TSLPR complex.

3. The bispecific antibody according to claim 1 or 2, wherein the epitope of human TSLP bound by one of the first binding fragment and the second binding fragment partially overlaps with the epitope of human TSLP bound by IL-7Rα.

4. The bispecific antibody according to any one of claims 1-3, wherein
the first binding fragment comprises HCDR1 as set forth in SEQ ID NO:29, HCDR2 as set forth in SEQ ID NO:30, HCDR3 as set forth in SEQ ID NO:31, LCDR1 as set forth in SEQ ID NO:32, LCDR2 as set forth in SEQ ID NO:33, and LCDR3 as set forth in SEQ ID NO:34; and/or
the second binding fragment comprises HCDR1 as set forth in SEQ ID NO:35, HCDR2 as set forth in SEQ ID NO:36, HCDR3 as set forth in SEQ ID NO:37, LCDR1 as set forth in SEQ ID NO:32, LCDR2 as set forth in SEQ ID NO:33, and LCDR3 as set forth in SEQ ID NO:34;
wherein the amino acid sequences of HCDRs and LCDRs are defined according to Kabat.

5. The bispecific antibody according to claim 4, wherein
the amino acid sequence of the heavy chain variable region of the first binding fragment is set forth in SEQ ID NO: 24, and the amino acid sequence of the light chain variable region of the first binding fragment is set forth in SEQ ID NO: 21; or
the amino acid sequence of the heavy chain variable region of the first binding fragment is set forth in SEQ ID NO: 24, and the amino acid sequence of the light chain variable region of the first binding fragment is set forth in SEQ ID NO: 28; and/or
the amino acid sequence of the heavy chain variable region of the second binding fragment is set forth in SEQ ID NO: 20, and the amino acid sequence of the light chain variable region of the second binding fragment is set forth in SEQ ID NO: 21; or
the amino acid sequence of the heavy chain variable region of the second binding fragment is set forth in SEQ ID NO: 20, and the amino acid sequence of the light chain variable region of the second binding fragment is set forth in SEQ ID NO: 28.

6. The bispecific antibody according to any one of claims 1-5, wherein
the bispecific antibody is an IgG1 antibody comprising a first heavy chain constant region and a second heavy chain constant region, wherein
the amino acids at positions 354 and 366 of the first heavy chain constant region are C and W, respectively, and the amino acids at positions 349, 366, 368 and 407 of the second heavy chain constant region are C, S, A and V, respectively; and/or
the amino acids at positions 234, 235 and 331 of the first heavy chain constant region and the second heavy chain constant region are F, E and S, respectively;
and the amino acid positions of antibody constant regions are determined according to EU numbering.

7. The bispecific antibody according to any one of claims 1-6, wherein
the forms of the first binding fragment and the second binding fragment are independently selected from a single-chain fragment variable (scFv) or a Fab fragment; preferably, both of the first binding fragment and the second binding fragment are Fab fragments; and/or
the first binding fragment and the second binding fragment have the same light chain variable region; preferably, the first binding fragment and the second binding fragment comprise the same light chain;
optionally, the bispecific antibody comprises the heavy chain as set forth in SEQ ID NO:38 and the light chain as set forth in one of SEQ ID NOs: 39 and 40; and/or
the bispecific antibody comprises the heavy chain as set forth in SEQ ID NO:41 and the light chain as set forth in one of SEQ ID NOs: 39 and 40.

8. A monoclonal antibody that binds to human TSLP, comprising HCDR1 as set forth in SEQ ID NO:29, HCDR2 as set forth in SEQ ID NO:30, HCDR3 as set forth in SEQ ID NO:31, LCDR1 as set forth in SEQ ID NO:32, LCDR2 as set forth in SEQ ID NO:33, and LCDR3 as set forth in SEQ ID NO:34;
wherein the amino acid sequences of HCDRs and LCDRs are defined according to Kabat;
preferably, the amino acid sequence of the heavy chain variable region of the monoclonal antibody is set forth in SEQ ID NO: 24, and the amino acid sequence of the light chain variable region of the monoclonal antibody is set forth in SEQ ID NO:21; or
the amino acid sequence of the heavy chain variable region of the monoclonal antibody is set forth in SEQ ID NO: 24, and the amino acid sequence of the light chain variable region of the monoclonal antibody is set forth in SEQ ID NO:28.

9. A pharmaceutical composition comprising the bispecific antibody of any one of claims 1-7, or the monoclonal antibody of claim 8, and a pharmaceutically acceptable excipient, diluent, or carrier.

10. The pharmaceutical composition according to claim 9 for use in the prevention or treatment of a TSLP-mediated disease; preferably, the TSLP-mediated disease is selected from the group consisting of asthma, scleroderma, systemic lupus erythematosus, rheumatoid arthritis, Churg-Strauss syndrome, Wegener's granulomatosis, allergic pneumonia, atopic dermatitis, rhinitis, Crohn's disease, psoriatic arthritis, chronic nephritis, Sjogren's syndrome, and multiple sclerosis.

11. Use of the bispecific antibody according to any one of claims 1-7, the monoclonal antibody according to claim 8, or the pharmaceutical composition according to claim 9 or 10 in the manufacture of a medicament for the prevention or treatment of a TSLP-mediated disease; preferably, the TSLP-mediated disease is selected from the group consisting of asthma, scleroderma, systemic lupus erythematosus, rheumatoid arthritis, Churg-Strauss syndrome, Wegener's granulomatosis, allergic pneumonia, atopic dermatitis, rhinitis, Crohn's disease, psoriatic arthritis, chronic nephritis, Sjogren's syndrome, and multiple sclerosis.

12. A method of preventing or treating a TSLP-mediated disease, comprising administering to a subject in need thereof the bispecific antibody according to any one of claims 1-7, the monoclonal antibody according to claim 8, or the pharmaceutical composition according to claim 9 or 10; preferably, the TSLP-mediated disease is selected from the group consisting of asthma, scleroderma, systemic lupus erythematosus, rheumatoid arthritis, Churg-Strauss syndrome, Wegener's granulomatosis, allergic pneumonia, atopic dermatitis, rhinitis, Crohn's disease, psoriatic arthritis, chronic nephritis, Sjogren's syndrome, and multiple sclerosis.
